# EUROPÄISCHE PATENTSCHRIFT

(11) **EP 0 538 193 B1**
(45) Veröffentlichungstag und Bekanntmachung des Hinweises auf die Patenterteilung: **04.12.1996**
(21) Anmeldenummer: 92810767.1
(22) Anmeldetag: 08.10.1992
(51) Int. Cl.: C07C 271/18, A61K 31/155, C07C 281/18

(54) **Kondensierte cycloaliphatische Amidinohydrozon-Salze als S-Adenosylmelthionin de carboxylase Inhibitoren**
Condensed cycloaliphatir-amidino-hydrazon salts as S-adenosylmethionin decarboxylase inhibitors
Sels d'amidinohydrones de composés cycloaliphatiques condensés comme inhibiteurs de S-adénosylméthionin décarboxylase

(30) Priorität: 16.10.1991 CH 3041/91
(43) Veröffentlichungstag der Anmeldung: 21.04.1993
(73) Patentinhaber: CIBA-GEIGY AG, 4002 Basel (CH)
(72) Erfinder: Stanek, Jaroslav, Dr., CH-4144 Arlesheim (CH); Frei, Jörg, Dr., CH-4434 Hölstein (CH); Caravatti, Giorgio, Dr., CH-4123 Allschwil (CH)

(56) Entgegenhaltungen:
- EP-A- 0 456 133

## Beschreibung

Die Erfindung betrifft Säureadditionssalze von Basen der Formel I 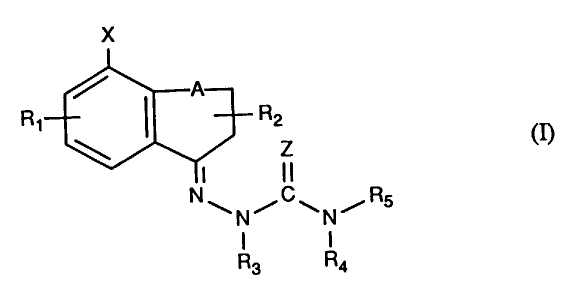 worin
A eine direkte Bindung bedeutet,
X für einen Rest -C(=NH)-NH₂ steht;
Z für NH steht; und
R₁, R₂, R₃, R₄ und R₅ jeweils Wasserstoff bedeuten;
mit einer Säure [PA], welche eine ein- oder mehrprotonige Säure ausgewählt aus Kohlensäure, Alkansäuren, die unsubstituiert oder ein bis mehrfach substituiert sind, ausser Ameisensäure, unsubstituierter Essigsäure, Lysin und Arginin, Alkensäuren, die unsubstituiert oder substituiert sind, ausser unsubstituierter Fumarsäure, Cycloalkylcarbonsäuren, Arylcarbonsäuren, Arylniederalkylcarbonsäuren, worin Niederalkyl Methyl oder Ethyl bedeutet und unsubstituiert oder substituiert ist, Aryl-C₂-C₄-alkenylcarbonsäuren, Heterocyclylcarbonsäuren, Alkansulfonsäuren, die unsubstituiert oder substituiert sind, ausser unsubstituierter Methansulfonsäure, aromatische Sulfonsäuren, Alkylschwefelsäuren, N-substituierten Sulfaminsäuren, organischen Säuren ohne Carboxy-, Sulfo-, Sulfat- oder Phosphogruppen, und aus Pyrophosphorsäure und Iodwasserstoff, bedeutet, Tautomere davon, ferner Verfahren zur Herstellung dieser Säureadditionssalze, pharmazeutische Präparate, die diese Säureadditionssalze enthalten, und die Verwendung dieser Säureadditionssalze zur Herstellung pharmazeutischer Präparate.

Der entsprechende Guanylrest, in Formel I als -N(R₃)-C(=Z)-NR₄R₅ dargestellt, kann auch in den tautomeren Formen -N=C(-ZH)-NR₄R₅, -N(R₃)-C(-ZH)=NR₅ oder -N(R₃)-C(-ZH)=NR₄ vorliegen.

Dem Fachmann ist das Vorliegen solcher und ähnlicher Tautomere geläufig. Alle diese Tautomere werden von der allgemeinen Formel I mit umfasst.

Die vor- und nachstehend verwendeten Allgemeinbegriffe haben im Rahmen der vorliegenden Anmeldung vorzugsweise die folgenden Bedeutungen:

Die folgenden Definitionen beziehen sich auf die Säuren [PA] ([PA] steht für "Protic Acid"):

Alkansäuren sind vor allem C₁-C₂₀-Alkansäuren mit Ausnahme von Ameisensäure und unsubstituierter Essigsäure, vorzugsweise C₂-C₇-Alkansäuren, wie Propionsäure, Buttersäure, Isobuttersäure, Pentansäure, Hexansäure oder Heptansäure, oder ferner Octansäure, Decansäure oder Dodecansäure, insbesondere Propionsäure oder Octansäure, wobei diese Säuren unsubstituiert oder ein bis mehrfach, insbesondere ein bis sechsfach, substituiert sind, vorzugsweise durch Hydroxy, entweder einfach, wie in Glykolsäure, Milchsäure oder 2-Hydroxybuttersäure, oder mehrfach, z. B. bis zu fünffach, wie in Gluconsäure oder Glucosemonocarbonsäure ("Glucoheptonic Acid"), durch Carboxy, z. B. in C₂-C₂₀-Alkandisäuren, vor allem C₂-C₇-Alkandisäuren, wie in Bernsteinsäure, oder ferner Adipinsäure, Pimelinsäure, Suberinsäure oder Azelainsäure, durch Hydroxy und Carboxy, wie in Äpfelsäure, Weinsäure, Zitronensäure, Glucarsäure oder Galactarsäure, Amino oder Amino und Carboxy und/oder ein oder zwei unabhängig aus Mercapto, Methylmercapto, Hydroxy, Phenyl, 4-Hydroxyphenyl, Naphthyl, Cyclohexyl, Imidazolyl oder Indolyl ausgewählte Reste, wie in Aminosäuren, wobei Lysin und Arginin ausgeschlossen sind, insbesondere Glutaminsäure oder Asparaginsäure in der (D)-, (L)- oder (D,L)-, vorzugsweise der (D)- oder (L)-Form, substituiertes Amino oder substituiertes Amino und Carboxy und/oder ein oder zwei unabhängig aus Mercapto (auch in oxidierter Form als das entsprechende Disulfid aus zwei Molekülen des entsprechenden Mercaptans), Methylmercapto, Hydroxy, Phenyl, 4-Hydroxyphenyl, Naphthyl, Cyclohexyl, Imidazolyl und Indolyl ausgewählte Reste, wie in Aminosäuren, z. B. in N-Mono- oder N,N-Diniederalkylaminosäuren, wie N-Methylglycin, oder in N-Niederalkanoylaminosäuren, wie Acetylaminoessigsäure (N-Acetylglycin), N-Acetylasparagin oder N-Acetylcystin, Oxo, wie in Brenztraubensäure oder Acetessigsäure, Phospho und Amino, wie in Phosphoserin, oder Phospho und Hydroxy, wie in 2- oder 3-Glycerophosphorsäure, Glucose-6-phosphorsäure, Glucose-1-phosphorsäure oder Fructose-1,6-bisphosphorsäure. Aminosäuren sind vor allem α-Aminosäuren, die in der (D)-, (L)- oder (D,L)-Form, vorzugsweise der (L)-oder (D)-Form, und bei Vorliegen weiterer Asymmetriezentren als weitere Isomere vorliegen können, z. B. ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH) (das auch in oxidierter Form vorliegen kann als Cystin), Phenylalanin (H-Phe-OH), Tyrosin (H-Tyr-OH), β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Asparaginsäure (H-Asp-OH), Asparagin (H-Asn-OH), Aminomalonsäure, Glutaminsäure (H-Glu-OH), Glutamin (H-Gln-OH), Histidin (H-HisOH), δ-Hydroxylysin, Omithin (α,δ-Diaminovaleriansäure), α,γ-Diaminobuttersäure und α,β-Diaminopropionsäure, und, sofern nichts anderes gesagt ist, aus Arginin (H-Arg-OH) und Lysin (H-Lys-OH). Besonders bevorzugte Aminosäuren sind Glycin, Serin, Cystin, Asparaginsäure und Glutaminsäure, vor allem Asparaginsäure und Glutaminsäure. Aminosäuren können bei der Definition von substituierten Alkansäuren auch weggelassen werden.

Alkensäuren sind beispielsweise C₂-C₁₀-Alkendisäuren, die unsubstituiert, wie Maleinsäure, oder substituiert sind, vorzugsweise durch Hydroxy, wie in Hydroxymaleinsäure (Tautomeres: Oxalessigsäure), oder Niederalkyl, z. B. Methyl, wie in Methylmaleinsäure. Unsubstituierte Fumarsäure ist ausgeschlossen.

Cycloalkylcarbonsäuren sind vorzugsweise C₄-C₁₂-Cycloalkylcarbonsäuren, worin der Cycloalkylrest mono-, bi- oder tricyclisch ist, vorzugsweise monocyclisch oder tricyclisch, z. B. Cyclopentyl, Cyclohexyl, Cycloheptyl oder Adamantyl, und beispielsweise unsubstituiert ist, wie in Cyclohexancarbonsäure oder Adamantancarbonsäure.

In Arylcarbonsäuren hat der Arylrest beispielsweise 6 bis 20, vorzugsweise 6 bis 14 Kohlenstoffatome, und ist z. B. ausgewählt aus Phenyl, 1- oder 2-Naphthyl und Indan, und ist unsubstituiert, wie in Benzoesäure, oder substituiert, vorzugsweise durch 1 bis 3 unabhängig aus Niederalkyl, wie Methyl, Halogen, wie Fluor, Chlor oder Brom, Hydroxy, Niederalkoxy, z. B. Methoxy, Phenoxy, Niederalkanoyloxy, wie Acetoxy, Amino und Carboxy ausgewählte Reste, wie in Salicylsäure, 1- oder 3-Hydroxynaphthyl-2-carbonsäure, 3,4,5-Trimethoxybenzoesäure, 2-Phenoxybenzoesäure, 2-Acetoxybenzoesäure, 4-Aminosalicylsäure oder Phthalsäure.

In Arylniederalkylcarbonsäuren ist Aryl wie zuletzt für Arylcarbonsäuren definiert und unsubstituiert oder wie dort definiert substituiert, und Niederalkyl ist Methyl oder Ethyl, wobei Niederalkyl unsubstituiert, wie in Phenylessigsäure, oder substituiert ist, z. B. durch Hydroxy, wie in Mandelsäure.

In Aryl-C₂-C₄-alkenylcarbonsäuren ist Aryl wie zuletzt für Arylcarbonsäuren definiert, wie in Zimtsäure.

Heterocyclylcarbonsäuren enthalten beispielsweise Heterocyclyl aus ein bis drei Ringen, vorzugsweise aus einem oder zwei Ringen, das gesättigt oder teilweise oder völlig ungesättigt, vorzugsweise gesättigt oder ungesättigt, ist, 5 bis 12 Ringatome, vorzugsweise 5 bis 7 Ringatome, hat, die aus Kohlenstoff und bis zu drei Heteroatomen ausgewählt sind, wobei vorzugsweise ein bis zwei Heteroatome vorliegen, insbesondere Sauerstoff, Stickstoff und/oder Schwefel, in erster Linie Sauerstoff und Stickstoff, wobei Heterocylyl z. B. Pyrrolyl, Imidazolyl, Pyrazolyl, Oxazolyl, Pyrazinyl, Pyrimidinyl, Indolyl, Chinolyl, Isochinolyl, Chinoxalinyl oder ein ganz oder teilweise gesättigtes Derivat dieser Reste oder Pyranyl oder Furanyl bedeutet, und unsubstituiert, wie in Nicotinsäure oder Isonicotinsäure, oder ein bis fünffach substituiert ist, vorzugsweise durch Hydroxy und/oder Hydroxyniederalkyl, z. B. Hydroxy oder Hydroxymethyl, wie in Glucuronsäure oder Galacturonsäure.

Alkansulfonsäuren sind vor allem C₂-C₂₀-Alkansulfonsäuren, vorzugsweise C₂-C₇-Alkansulfonsäuren, wie Ethansulfonsäure, welche unsubstituiert oder substituiert sind, vorzugsweise durch ein bis zwei Reste ausgewählt aus Hydroxy und Sulfo, wie in 2-Hydroxyethansulfonsäure oder Alkandisulfonsäuren, z. B. Niederalkandisulfonsäuren, wie Ethan-1,2-disulfonsäure. Methansulfonsäure ist ausgeschlossen.

In aromatischen Sulfonsäuren ist der aromatische Rest beispielsweise Aryl wie für Arylcarbonsäuren definiert und unsubstituiert, wie in Benzolsulfonsäure oder 2-Naphthalinsulfonsäure, oder wie dort substituiert, insbesondere durch Niederalkyl, z. B. Methyl, wie in 2-, 3- oder 4-Methylbenzolsulfonsäure, oder durch einen weiteren Sulfonylrest, wie in 1,3-Benzolsulfonsäure oder Naphthalin-1,5-disulfonsäure, vor allem wie in 1,5-Naphthalindisulfonsäure.

Alkylschwefelsäuren sind vor allem C₁-C₂₀-Alkylschwefelsäuren, insbesondere Niederalkylschwefelsäuren, wie Methylschwefelsäure oder Ethylschwefelsäure, oder Dodecylschwefelsäure.

N-substituierte Sulfaminsäuren sind beispielsweise N-Cycloalkylsulfaminsäuren, worin Cycloalkyl vorzugsweise C₄-C₁₂-Cycloalkyl bedeutet und der Cycloalkylrest mono-, bi-oder tricyclisch ist, z. B. Cyclopentyl, Cyclohexyl, Cycloheptyl oder Adamantyl, vorzugsweise monocyclisch, wie in N-Cyclohexylsulfaminsäure, oder N-Alkylsulfaminsäuren, vorzugsweise N-Niederalkylsulfaminsäure, wie Methyl-, Ethyl- oder Propylsulfaminsäure.

Organische Säuren ohne Carboxy-, Sulfo-, Sulfat- oder Phosphogruppen enthalten beispielsweise saure Hydroxygruppen, wie in Ascorbinsäure.

Wichtige Säuren sind Kohlensäure, Propionsäure, Octansäure, Decansäure, Dodecansäure, Glykolsäure, Milchsäure, 2-Hydroxybuttersäure, Gluconsäure, Glucosemonocarbonsäure, Bernsteinsäure, Adipinsäure, Pimelinsäure, Suberinsäure, Azelainsäure, Äpfelsäure, Weinsäure, Zitronensäure, Glucarsäure, Galactarsäure, Glutaminsäure, Asparaginsäure, N-Methylglycin, Acetylaminoessigsäure, N-Acetylasparagin, N-Acetylcystin, Brenztraubensäure, Acetessigsäure, Phosphoserin, 2- oder 3-Glycerophosphorsäure, Glucose-6-phosphorsäure, Glucose-1-phosphorsäure, Fructose1,6-bisphosphorsäure, Maleinsäure, Hydroxymaleinsäure, Methylmaleinsäure, Cyclohexancarbonsäure, Adamantancarbonsäure, Benzoesäure, Salicylsäure, 1- oder 3-Hydroxynaphthyl-2-carbonsäure, 3,4,5-Trimethoxybenzoesäure, 2-Phenoxybenzoesäure, 2-Acetoxybenzoesäure, 4-Aminosalicylsäure, Phthalsäure, Phenylessigsäure, Mandelsäure, Zimtsäure, Nicotinsäure, Isonicotinsäure, Glucuronsäure, Galacturonsäure, Ethansulfonsäure, 2-Hydroxyethansulfonsäure, Ethan-1,2-disulfonsäure, Benzolsulfonsäure, 2-Naphthalinsulfonsäure, 1,5-Naphthalindisulfonsäure, 2-, 3- oder 4-Methylbenzolsulfonsäure, Methylschwefelsäure, Ethylschwefelsäure, Dodecylschwefelsäure, N-Cyclohexylsulfaminsäure, Methyl-, Ethyl- oder Propylsulfaminsäure, oder Ascorbinsäure.

Sehr wichtige Säuren sind Octansäure, Bernsteinsäure, Adipinsäure, Salicyclsäure, Benzolsulfonsäure, 1,5-Naphthalinsulfonsäure oder N-Cyclohexylsulfaminsäure, ganz besonders Salicylsäure oder, in erster Linie, Adipinsäure oder Benzolsulfonsäure.

Sehr wichtige Säuren sind in gleicher Weise Weinsäure, insbesondere L-Weinsäure, Milchsäure oder Zitronensäure.

Die genannten Säuren können, insbesondere sofern sie mehrere saure Gruppen mit unterschiedlicher Acidität enthalten, welche Protonen dissoziieren können, auch als Mischsalze mit Kationen, z. B. Alkalimetallkationen, wie Natrium- oder Kaliumionen, Alkalimetallsalze, wie Magnesiumsalze, oder Zinksalze vorliegen, wobei vor der Umsetzung zu den genannten Mischsalzen mindestens noch ein dissoziierbares Proton an der Säurekomponente enthalten ist, oder sie können in den gebildeten Salzen in einer Form vorliegen, wo nicht alle Protonen freigesetzt sind, mindestens jedoch ein Proton auf die jeweilige Base der Formel I übertragen ist. So kann beispielsweise Kohlensäure als Bicarbonatsalz, wie Natrium- oder Kaliumbicarbonat, eingesetzt werden.

Die Säureadditionssalze von einer Base der Formel I mit einer Säure [PA] können auch als Hydrate vorliegen. Kristalle können auch andere zur Kristallisation verwendete Lösungsmittel einschliessen.

Je nach den strukturellen Gegebenheiten können die Säureadditionssalze der vorliegenden Erfindung in Form von Isomerengemischen oder von reinen Isomeren vorliegen.

Das Ziel der vorliegenden Erfindung ist, neue Säureadditionssalze von pharmakologisch verwendbaren Verbindungen zur Verfügung zu stellen, die eine gute Löslichkeit in physiologischen Flüssigkeiten und/oder Flüssigkeiten, die physiologischen Flüssigkeiten ähnlich sind, wie physiologische Kochsalzlösung, Mannitol-Lösung oder Phosphatpuffer, und/oder gute Resorbierbarkeit bei enteraler, wie oraler, Verabreichung aufweisen, beispielsweise durch Bildung von Ionenpaaren, wie lipophilen Ionenpaaren.

Die erfindungsgemässen Säureadditionssalze weisen wertvolle pharmakologisch verwertbare Eigenschaften auf. Insbesondere zeigen sie eine starke, spezifische Hemmwirkung auf das Enzym S-Adenosylmethionindecarboxylase (SAMDC). SAMDC spielt als ein Schlüsselenzym eine wichtige Rolle bei der Polyaminsynthese, die in praktisch allen Zellen von Säugetieren, einschliesslich Menschen, abläuft. Durch SAMDC wird die Polyaminkonzentration in der Zelle reguliert. Eine Hemmung des Enzyms SAMDC hat eine Verringerung der Polyaminkonzentration zur Folge. Da eine Verringerung der Polyaminkonzentration eine Hemmung des Zellwachstums bewirkt, ist es möglich, durch Verabreichung von SAMDC-hemmenden Substanzen das Wachstum sowohl von eukaryotischen als auch prokaryotischen Zellen zu hemmen und sogar Zellen abzutöten oder das Einsetzen der Zelldifferenzierung zu hemmen.

Die Hemmung des Enzyms SAMDC kann z.B. mit der Methode von H.G. Williams-Ashmann und A. Schenone, Biochem. Biophys. Res.Communs. 46, 288 (1972) nachgewiesen werden. Die Säureadditionssalze der Erfindung weisen hier IC₅₀-Werte von minimal 0.005 µM und weniger auf.

Ein weiterer Vorteil der erfindungsgemässen Säureadditionssalze besteht darin, dass sie im Vergleich zu ihrer starken Hemmwirkung auf SAMDC nur in geringem Masse die Diaminoxidase inhibieren und gut verträglich sind. Die Hemmung der Diaminoxidase ist nach J. Jaenne und D.R. Morris, Biochem. J. 218, 974 (1984) ungünstig, da sie zur Akkumulation von Putrescin und einer indirekten SAMDC-Aktivierung führen kann.

Daher sind die Säureadditionssalze der Formel I z.B. nützlich zur Behandlung benigner und maligner Tumoren. Sie können Tumorregressionen bewirken und ferner die Verbreitung von Tumorzellen mit der Folge einer Metastasenbildung sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Als selektive SAMDC-Hemmer können die Säureadditionssalze von Basen der Formel I mit einer Säure [PA] allein oder auch in Kombination mit anderen pharmakologisch wirksamen Substanzen angewendet werden. Zu denken ist z.B. an eine Kombination mit (a) Inhibitoren anderer Enzyme der Polyaminbiosynthese, z.B. Ornithindecarboxylasehemmern, (b) Inhibitoren der Proteinkinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Die Erfindung betrifft in allererster Linie die Säureadditionssalze von Basen der Formel I, worin A eine direkte Bindung bedeutet, X für einen Rest -C(=NH)-NH₂ steht; Z für NH steht; und R₁, R₂, R₃, R₄ und R₅ jeweils Wasserstoff bedeuten; und [PA] aus den oben genannten Säuren ausgewählt ist, oder vorzugsweise eine der oben als wichtig, beispielsweise eine der als sehr wichtig definierten Säuren z. B. eine der Säuren ausgewählt aus N-Cyclohexylsulfaminsäure, Octansäure, Salicylsäure oder Benzolsulfonsäure, wie Salicylsäure, bedeutet.

Die Erfindung betrifft in allererster Linie auch die Säureadditionssalze von Basen der Formel I, worin A eine direkte Bindung bedeutet, X für einen Rest -C(=NH)-NH₂ steht; Z für NH steht; und R₁, R₂, R₃, R₄ und R₅ jeweils Wasserstoff bedeuten; und [PA] vorzugsweise eine der oben als sehr wichtig definierten Säuren, insbesondere ausgewählt aus Weinsäure, in erster Linie L-Weinsäure, Milchsäure und Zitronensäure, bedeutet.

Am stärksten bevorzugt sind die in den Beispielen genannten Säureadditionssalze von Basen der Formel I mit einer Säure [PA].

Die Säureadditionssalze von Basen der Formel I mit einer Säure [PA] werden nach an sich bekannten Verfahren hergestellt, beispielsweise indem man
a) eine Base der Formel I 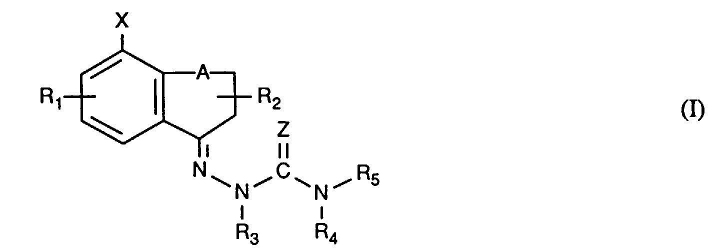 worin die Reste die für Säureadditionssalze von Basen der Formel I genannten Bedeutungen haben, mit einer Säure [PA], wobei [PA] die oben genannten Bedeutungen hat, umsetzt, oder
b) eine Verbindung der Formel 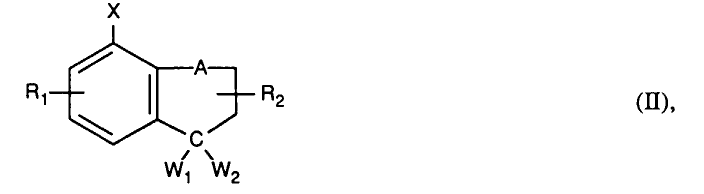 worin die Gruppe CW₁W₂ für Carbonyl, funktionell abgewandeltes Carbonyl oder geschütztes Carbonyl steht und A, X, R₁ und R₂ die für Säureadditionssalze der Formel I genannten Bedeutungen haben, mit einem Amin der Formel 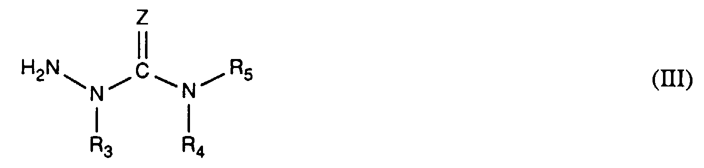 worin Z, R₃, R₄ und R₅ die für Säureadditionssalze von Basen der Formel I genannten Bedeutungen haben, in Gegenwart einer Säure [PA] mit den genannten Bedeutungen kondensiert, oder
c) in einer Verbindung der Formel 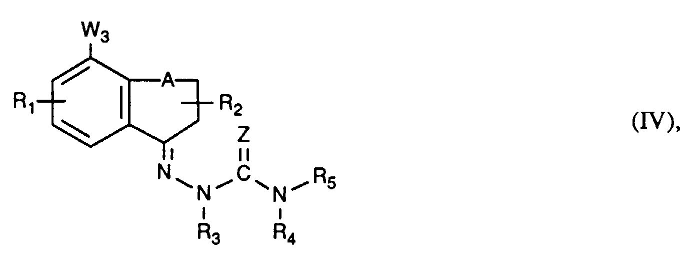 worin W₃ einen Rest, der in eine Gruppe X in einer Base der Formel I überführt werden kann, bedeutet und A, Z, R₁, R₂, R₃, R₄ und R₅ wie unter Formel I definiert sind, den Rest W₃ in Gegenwart einer Säure [PA] mit den oben genannten Bedeutungen in die Gruppe X überführt; oder
d) ein beliebiges Säureadditionssalz von einer Base der Formel I mit einer Säure, die nicht unter die Definition von [PA] fällt, in ein Säureadditionssalz von einer Base der Formel I mit einer Säure [PA] mit den oben genannten Bedeutungen umwandelt;
und, wenn gewünscht, ein erhaltenes Säureadditionssalz einer Base der Formel I mit einer Säure [PA] in ein anderes Säureadditionssalz einer Base der Formel I mit einer Säure [PA] umwandelt, und/oder, wenn gewünscht, Isomerengemische auftrennt in die einzelnen Isomeren.

In der folgenden näheren Beschreibung der Verfahren a)-d) haben die Symbole A, X, Y, Z, R₁-R₉ und [PA] jeweils die bei der Definition der Säureadditionssalze von Basen der Formel I mit Säuren [PA] angegebenen Bedeutungen, sofern nichts anderes angegeben ist.

### Verfahren a)

Die Umsetzung einer Base der Formel I mit einer Säure [PA] zu dem entsprechenden Säureadditionssalz erfolgt nach an sich bekannten Methoden zur Bildung von Säureadditionssalzen basischer Verbindungen.

Die Umsetzung zum Säureadditionssalz geschieht beispielsweise in Lösungsmitteln, insbesondere in organischen Lösungsmitteln, vor allem in polaren organischen Lösungsmitteln, in erster Linie in Estern, z. B. Niederalkanoylniederalkylestern, wie Essigsäureethylester, in Amiden, z. B. N,N-Diniederalkyl-niederalkanoylamiden, wie Dimethylformamid, in Alkoholen, z. B. Hydroxyniederalkanen, wie Methanol, Ethanol, Ethylenglykol oder Glycerin, oder Arylalkoholen, wie Phenolen, z. B. Phenol, oder in Dimethylsulfoxid, in Abwesenheit oder Gegenwart von Wasser, vorzugsweise in Abwesenheit von Wasser. Besonders bevorzugt ist die Umsetzung in Alkoholen, wie den zuletzt genannten Hydroxyniederalkanen.

Die Umsetzung geschieht beispielsweise in freier Lösung, kann aber auch *a*n chromatographischen Säulen, z. B. durch Gelfiltration oder an Ionenaustauschern, oder über semipermeable Membranen durch osmotische Vorgänge, erfolgen.

Die Umsetzung erfolgt bei Temperaturen zwischen dem Gefrier- und dem Siedepunkt der betreffenden Lösungen, vorzugsweise zwischen 0 und 50 °C, insbesondere zwischen 20 und 40 °C, z. B. bei Raumtemperatur, in Gegenwart oder Abwesenheit eines Schutzgases, wie Stickstoff oder Argon.

Die Verbindungen der Formel I und die Säuren [PA] werden in geeigneten molaren Verhältnissen eingesetzt, oder die Säure [PA] wird im Überschuss eingesetzt. Bevorzugt werden die Einzelkomponenten in der molaren Relation eingesetzt, die dem Verhältnis der Molarität der Base der Formel I und der Säure [PA] in den erfindungsgemässen Säureadditionssalzen entspricht.

Die gebildeten Salze fallen beispielsweise, gegebenenfalls erst nach Abkühlung, von selbst aus, oder sie werden durch Zugabe von Lösungsmitteln, insbesondere von unpolaren Lösungsmitteln, z. B. Ethern, wie Diethylether, oder von Wasser ausgefällt und/oder durch teilweises oder völliges Eindampfen gewonnen.

### Verfahren b)

Als funktionell abgewandeltes bzw. geschütztes Carbonyl CW₁W₂ sind beispielhaft zu nennen: Diniederalkoxymethyl, C₁-C₂-Alkylendioxymethyl, Dihalogenmethyl, Diniederalkylthiomethyl oder C₁-C₂-Alkylendithiomethyl.

Bevorzugt liegt die Gruppe CW₁W₂ in den Verbindungen der Formel II als freies Carbonyl vor.

Die Kondensationsreaktion gemäss Verfahren (b) erfolgt unter den an sich bekannten Bedingungen bei der Bildung von Hydrazonen und wird in Gegenwart einer Säure [PA] durchgeführt, die zugleich katalytisch wirksam ist. [PA] liegt vorzugsweise in einer Menge vor, bei der die Protonierung der reagierenden Aminogruppe in der Verbindung der Formel III nur in so geringem Ausmass erfolgt, dass sie noch reaktionsfähig ist. Besonders bevorzugt werden hierzu die Ausgangsverbindungen, sofern sie salzbildende Gruppen enthalten, als Salze der Säure [PA] eingesetzt, die gegebenenfalls in situ hergestellt werden, entweder aus den freien Verbindungen oder Salzen leicht flüchtiger Säuren, wie Halogenwasserstoffsäuren, z. B. HBr oder HCl, Ameisensäure, Essigsäure oder Kohlensäure (als Carbonat- oder Bicarbonat-Salz), wobei die Reaktion beispielsweise auch in Gegenwart eines katalytisch wirksamen kleinen Überschusses der Säure [PA] erfolgen kann. Als Lösungsmittel findet vorzugsweise eines der unter Verfahren a) genannten Lösungsmittel Verwendung, insbesondere ein wässriges Lösungsmittel, wie Wasser, bei bevorzugten Temperaturen von 20 ° C bis zum Siedepunkt des betreffenden Reaktionsgemisches, insbesondere beim Siedepunkt des betreffenden Gemisches, in An- oder Abwesenheit eines Schutzgases, wie Stickstoff oder Argon. Für Verbindungen der Formel II sind solche geschützte Carbonylgruppen CW₁W₂ geeignet, die unter den Bedingungen der Kondensation in freies Carbonyl übergehen.

Zur Herstellung von Säureadditionssalzen aus einer Base der Formel I, worin R₅ Amino bedeutet, und einer Säure [PA] empfiehlt es sich, die Verbindung der Formel III im Ueberschuss einzusetzen.

### Verfahren c)

In den Zwischenprodukten der Formel IV bedeutet W₃ z.B. freies oder funktionell abgewandeltes Carboxy, insbesondere Halogencarbonyl, Cyano, Imino-niederalkoxycarbonyl, Imino-niederalkylthiolcarbonyl oder Thiocarbamoyl.

Die Gruppe W₃ in einer Verbindung der Formel IV kann bei der Herstellung von Amidinen der Formel I z. B. bedeuten: ein Säureadditionssalz eines Iminoniederalkylesters (=̂ Iminoniederalkylether) oder Imino-niederalkylthiolesters mit einer der genannten Säuren, z. B. -C(=NH)-OC₂H₅ · [PA] bzw. -C(=NH)-SC₂H₅ · [PA], ferner Thiocarbamoyl oder Cyano.

Durch die Umsetzung eines Imino-niederalkylesters der Formel IV (als Salz einer der Säuren [PA], wie oben definiert) mit Ammoniak erhält man die unsubstituierten Säureadditionssalze der entsprechenden Amidine gemäss Formel I.

Die Carbonsäureesterimide erhält man z.B. durch säurekatalysierte Anlagerung von Alkoholen an die Nitrile der Formel IV. Aus den Esterimiden erhält man die Säureadditionssalze der Amide der Formel I im Sinne einer Pinner-Spaltung durch thermischen Zerfall der Esterimid-Salze mit Säuren [PA] bei Temperaturen oberhalb von etwa 80°C.

Säureadditionssalze von Basen der Formel I mit Säuren [PA] können auch durch Umsetzung von Verbindungen der Formel IV oder deren Säureadditionssalzen mit einer der oben genannten Säuren [PA], worin W₃ den Rest -C(=S)-NH₂ bedeutet, unter S-Al-kylierung, z.B. mit Triniederalkyloxonium-tetrafluoroborat, und anschliessender Umsetzung mit einem Ammoniaksalz einer der genannten Säuren [PA] erhalten werden.

Die unter c) angeführten Reaktionen können, soweit nichts anderes angegeben ist, unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. bei Raumtemperatur oder beim Siedepunkt des verwendeten Lösungsmittels in dem jeweiligen Reaktionsgemisch, unter atmosphärischem Druck oder in einem geschlossenen Gefäss, gegebenenfalls unter Druck, und/oder in einer inerten Atmosphäre, z.B. unter einer Stickstoffatmosphäre.

### Verfahren d)

Die Umsetzung eines beliebigen Säureadditionssalzes von einer Base der Formel I mit einer Säure, die nicht unter die Definition von [PA] fällt, geschieht nach den üblichen Methoden zur Umwandlung von Salzen.

Zu den Säuren, die nicht unter die Definition von [PA] fallen, gehören sämtliche weitere Protonsäuren, beispielsweise nicht unter die Definition von [PA] fallende organische Säuren, wie Ameisensäure, Essigsäure, Methansulfonsäure, oder anorganische Säuren, wie Schwefelsäure, Halogenwasserstoffsäuren, wie HF, HCI, HBr oder HI, ferner Stickstoffwasserstoffsäure oder Phosphorsäure. Besonders bevorzugt sind die Salze von Halogenwasserstoffsäuren.

Die Umsetzung derartiger Salze von Säuren, die nicht unter die Definition von [PA] fallen, zum Säureadditionssalz einer Säure [PA] geschieht beispielsweise in Lösungsmitteln, insbesondere in organischen Lösungsmitteln, vor allem in polaren organischen Lösungsmitteln, in erster Linie in Estern, z. B. Niederalkanoylniederalkylestern, wie Essigsäureethylester, in Amiden, z. B. N,N-Diniederalkyl-niederalkanoylamiden, wie Dimethylformamid, in Alkoholen, z. B. Hydroxyniederalkanen, wie Methanol, Ethanol, Ethylenglykol oder Glycerin, oder Arylalkoholen, wie Phenolen, z. B. Phenol, oder in Dimethylsulfoxid, in Abwesenheit oder Gegenwart von Wasser, vorzugsweise in Abwesenheit von Wasser. Besonders bevorzugt ist die Umsetzung in Alkoholen, wie den zuletzt genannten Hydroxyniederalkanen.

Die Umsetzung kann auch über die freien Basen der Formel I erfolgen, die beispielsweise hergestellt werden, indem man das als Ausgangsmaterial verwendete Säuresalz von einer Base der Formel I mit einer Säure, die nicht unter die Definition von [PA] fällt, mit Hilfe einer Base, z. B. einer Hydroxybase, wie einem Alkalihydroxid, z. B. NaOH oder KOH, in wässriger Lösung in An- oder Abwesenheit eines organischen Lösungsmittels, wie unter a) definiert, in die freie Base überführt; die anschliessende Umwandlung der freien Base erfolgt beispielsweise wie unter Verfahren a) beschrieben.

Die Verbindungen der Formel I und die Säuren [PA] werden bei den genannten Umsetzungen in geeigneten molaren Verhältnissen eingesetzt, oder die Säure [PA] wird im Überschuss eingesetzt. Bevorzugt werden die Einzelkomponenten in der molaren Relation eingesetzt, die dem Verhältnis der Molarität der Base der Formel I und der Säure [PA] in den erfindungsgemässen Säureadditionssalzen entspricht.

Die Umsetzung erfolgt bei Temperaturen zwischen dem Gefrier- und dem Siedepunkt der betreffenden Lösungen, vorzugsweise zwischen 0 und 50 °C, insbesondere zwischen 20 und 40 °C, z. B. bei Raumtemperatur, in Gegenwart oder Abwesenheit eines Schutzgases, wie Stickstoff oder Argon.

Die gebildeten Salze fallen beispielsweise, gegebenenfalls erst nach Abkühlung, von selbst aus, oder sie werden durch Zugabe von Lösungsmitteln, insbesondere von unpolaren Lösungsmitteln, z. B. Ethern, wie Diethylether, oder von Wasser ausgefällt und/oder durch teilweises oder völliges Eindampfen gewonnen.

### Zusätzliche Verfahrensmassnahmen

Säureadditionssalze von einer Base der Formel I mit einer Säure [PA] können in Säureadditionssalze von anderen Säuren [PA] überführt werden, wobei an sich bekannte Verfahren Verwendung finden.

Dies kann beispielsweise geschehen, indem man die Säure [PA] des Säureadditionssalzes umwandelt.

Beispielsweise kann die Säurekomponente [PA] ausgetauscht werden, entweder direkt durch Überführung in Gegenwart der einzuführenden Säure [PA], die z. B. im Überschuss eingesetzt werden kann, oder indirekt durch Überführung der im als Ausgangsmaterial verwendeten Säureadditionssalz enthaltenen Base der Formel I in die freie Base, beispielsweise durch Umsetzung des als Ausgangsmaterial verwendeten Säureadditionssalzes der Base der Formel I mit einer Base, insbesondere einer Hydroxybase, wie einem Alkalihydroxid, z. B. NaOH oder KOH, in wässriger Lösung in An- oder, vorzugsweise, Abwesenheit eines organischen Lösungsmittels, wie unter a) definiert, in die freie Base und anschliessende Umwandlung der freien Base gemäss Verfahren a) in ein anderes Säureadditionssalz der Formel I, ferner durch Dialyse, mittels Ionenaustauschern oder durch Gelchromatographie.

Erfindungsgemäss erhältliche Gemische von Isomeren können in an sich bekannter Weise in die einzelnen Isomeren aufgetrennt werden.

Im Verfahren der vorliegenden Erfindung werden vorzugsweise solche Ausgangsstoffe eingesetzt, die zu den eingangs als besonders wertvoll beschriebenen Säureadditionssalzen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

### Pharmazeutische Präparate:

Die vorliegende Erfindung betrifft ebenfalls pharmazeutische Präparate, die als Wirkstoff eines der pharmakologisch wirksamen Säureadditionssalze von einer Base der Formel I mit einer Säure [PA] enthalten. Besonders bevorzugt sind Präparate zur enteralen, insbesondere oralen, sowie zur parenteralen Verabreichung. Die Präparate enthalten den Wirkstoff allein oder vorzugsweise zusammen mit mindestens einem pharmazeutisch anwendbaren Trägermaterial. Die Dosierung des Wirkstoffes hängt von der zu behandelnden Krankheit, sowie von der Spezies, deren Alter, Gewicht und individuellem Zustand, sowie von der Applikationsweise ab.

Die pharmazeutischen Präparate enthalten von etwa 0,1 % bis etwa 95 % der Wirksubstanz, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragees, Tabletten oder Kapseln, enthalten von etwa 1 mg bis etwa 500 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls nach Zugabe von zusätzlichen Hilfsstoffen, zu Tabletten oder Dragée-Kernen verarbeiten.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat.

Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/ oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, welche den Wirkstoff z.B. in suspendierter Form und in einer Konzentration von ca. 0,1 % bis 10 % , vorzugsweise ca. 1 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyehylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann die Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden. Eine isotonische Lösung zur Infusion kann insbesondere durch Zugabe von geeigneten Salzen, wie NaCl, von Puffern, wie Phosphatpuffer, z. B. mit Natrium als Gegenion, und/oder von Zuckeralkoholen, wie Mannitol, hergestellt werden, wobei gegebenenfalls auch weitere der genannten Hilfsstoffe zugegen sein können.

Lösungen, wie sie z.B. für die parenterale Verabreichung verwendet werden, können auch als Infusionslösungen angewandt werden.

Die Säureadditionssalze von Basen der Formel I mit einer Säure [PA], wie oben definiert, ermöglichen ebenfalls ein Verfahren zur Behandlung der oben genannten, insbesondere durch fehlende S-Adenosylmethionindecarboxylase-Hemmung bedingten Krankheitszustände, die auf Behandlung mit einem Hemmer der S-Adenosylmethionindecarboxylase ansprechen. Die Säureadditionssalze der vorliegenden Erfindung können prophylaktisch oder therapeutisch, insbesondere in Mengen, die zur Hemmung der S-Adenosylmethionindecarboxylase geeignet sind, verabreicht werden, wobei man sie vorzugsweise in Form von pharmazeutischen Präparaten verwendet. Dabei wird bei einem Körpergewicht von etwa 70 kg eine, insbesondere gegen die genannten Erkrankungen wirksame, tägliche Dosis von etwa 1 mg bis etwa 1000 mg, vorzugsweise von etwa 25 bis 100 mg oral bzw. 2 bis 50 mg parenteral, eines Säureadditionssalzes der vorliegenden Erfindung verabreicht, z. B. an einen Warmblüter, wie einen Menschen, der einer derartigen Behandlung bedarf, z. B., weil sie an einer Protozoainfektion oder an Tumoren leiden.

Die Erfindung betrifft auch eine pharmazeutische Zusammensetzung, die geeignet ist zur Verabreichung an einen Säuger, z. B. Menschen, zur Vermeidung oder Behandlung einer Erkrankung, die auf eine Behandlung mit einem Hemmer der S-Adenosylmethionindecarboxylase anspricht, insbesondere einer Tumorerkrankung oder einer Protozoainfektion, umfassend eine zur Hemmung der S-Adenosylmethionindecarboxylase wirksame Menge eines Säureadditionssalzes der Formel I, oder Tautomere davon, und ein pharmazeutisch annehmbares Trägermaterial.

Die Erfindung betrifft auch ein erfindungsgemässes Säureadditionssalz zur Anwendung in einem Verfahren zur therapeutischen Behandlung des tierischen oder menschlichen Körpers, insbesondere zur Verwendung bei der Behandlung von Krankheiten, die auf eine Hemmung des Enzyms S-Adenosylmethionindecarboxylase ansprechen, sowie dessen Verwendung zur Herstellung pharmazeutischer Präparate für die Behandlung von Krankheiten, die auf eine Hemmung des Enzyms S-Adenosylmethionindecarboxylase ansprechen.

### Ausgangsverbindungen:

Die Protonensäuren [PA] sind bekannt, nach an sich bekannten Verfahren herstellbar oder auch kommerziell erhältlich.

Die Ausgangsverbindungen der Formel I werden nach an sich bekannten Verfahren beispielsweise wie folgt hergestellt, indem man
(i) eine Verbindung der Formel II, wie unter Verfahren a) definiert, mit einem Amin der Formel III, wie unter Verfahren a) definiert, kondensiert, oder
(ii) in einer Verbindung der Formel IV, wie unter Verfahren c) definiert, den Rest W₃ in die Gruppe X überführt,
und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

In der folgenden näheren Beschreibung der Verfahren i)-ii) haben die in Formeln vorkommenden Symbole A, X, Y, Z und R₁-R₉ sowie [PA] jeweils die für Säureadditionssalze von Basen der Formel I mit Säuren [PA] angegebenen Bedeutungen, sofern nichts anderes angegeben ist.

### Verfahren (i):

Als funktionell abgewandeltes bzw. geschütztes Carbonyl CW₁W₂ sind beispielhaft zu nennen: Diniederalkoxymethyl, C₁-C₂-Alkylendioxymethyl, Dihalogenmethyl, Diniederalkylthiomethyl oder C₁-C₂-Alkylendithiomethyl.

Bevorzugt liegt die Gruppe CW₁W₂ in den Verbindungen der Formel II als freies Carbonyl vor.

Die Kondensationsreaktion gemäss Verfahren (a) erfolgt unter den an sich bekannten Bedingungen bei der Bildung von Hydrazonen. Sie ist bevorzugt durch Säure katalysiert. In Verbindungen der Formel II sind solche geschützte Carbonylgruppen CW₁W₂ geeignet, die unter den Bedingungen der Kondensation in freies Carbonyl übergehen.

Zur Herstellung von Verbindungen der Formel I, worin R₅ Amino bedeutet, empfiehlt es sich, die Verbindung der Formel III im Ueberschuss einzusetzen.

Die Zwischenprodukte der Formel I werden z.B. dadurch erhalten, dass man eine Verbindung der Formel V 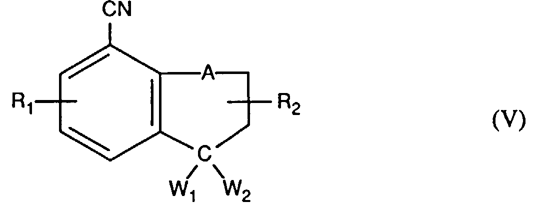 zunächst durch Behandlung mit Schwefelwasserstoff in das entsprechende Thiocarboxamid [-C(=S)-NH₂] überführt. Letzteres kann auch auf anderem Wege ausgehend vom analogen Carboxamid [-C(=O)-NH₂], z.B. durch Umsetzung mit dem Lawesson-Reagens [2,4-Bis-(4-methoxyphenyl)-2,4-dithioxo-1,3,2,4-dithiadiphosphetan], erhalten werden. Die Thiocarboxamide werden, z.B. mit Niederalkyliodid oder Triniederalkyloxoniumtetrafluoroborat, S-alkyliert und damit in Imino-niederalkylthiolester-Hydroiodide [-C(=NH)S-Alkyl·HI] bzw. -tetrafluoroborate überführt, welche sich leicht durch Umsetzung mit Ammoniak in die gewünschten Carboximidamide der Formel I überführen lassen [vgl. S. Patai (Ed.), The Chemistry of Amidines and Imidates, Wiley, London etc. 1975, S. 303-304].

Die Herstellung der Carboxamide der Formel II aus den Cyanoverbindungen der Formel V verläuft analog der unten beim Verfahren (ii) beschriebenen Herstellung von Carboxamiden der Formel I aus Cyanoverbindungen der Formel IV und ist dort detailliert beschrieben.

Eine weitere Möglichkeit zur Herstellung der Verbindungen der Formel II besteht darin, dass man eine Verbindung der Formel V, worin die Gruppe CW₁W₂ wie unter Formel II definiert ist, z.B. mit Ethanol und Salzsäure in z.B. Chloroform oder Diethylether behandelt, wobei das entsprechende Iminoethylester-Hydrochlorid gebildet wird, welches z.B. durch Umsetzung mit Ammoniak und z.B. Methanol in das gewünschte Carboximidamid der Formel II überführt werden kann.

Die Ausgangsverbindungen der Formel V sind an sich bekannt oder werden analog zu den bekannten Verbindungen hergestellt.

Die Verbindungen der Formel V lassen sich z.B. durch intramolekulare Friedel-Crafts-Acylierung von ω-Phenylniederalkansäuren der Formel VI, 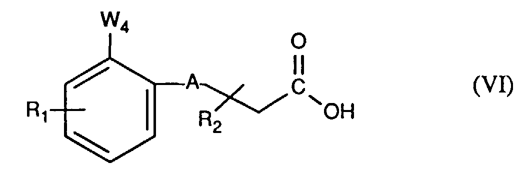 worin W₄ Cyano oder eine Cyanovorstufe bedeutet, oder Säurederivaten davon, z.B. Säurechloriden oder Säureanhydriden, herstellen. Als Katalysatoren können bei freien Säuren z.B. Polyphosphorsäure und bei Säurechloriden oder -anhydriden z.B. AlCl₃ verwendet werden.

Bevorzugt werden bei dieser Reaktion Verbindungen der Formel VI eingesetzt, worin W₄ nicht Cyano, sondern eine Cyanovorstufe, z.B. Halogen, insbesondere Brom, oder geschütztes Amino, z.B. Acetylamino, bedeutet. Nach dem Cyclisierungsschnitt können dann die Cyanovorstufen in an sich bekannter Weise in Cyano überführt werden, z.B. Brom durch Umsetzung mit Kupfer(I)cyanid oder Acetylamino durch Abspaltung der Acetylschutzgruppe, Diazotierung und Umsetzung mit Kupfer(I)cyanid.

Verbindungen der Formel V, worin die Gruppe CW₁W₂ Carbonyl bedeutet, lassen sich ferner z.B. durch Oxidation, z.B. mit Chromtrioxid (CrO₃), aus den entsprechenden Nichtcarbonylverbindungen der Formel VII 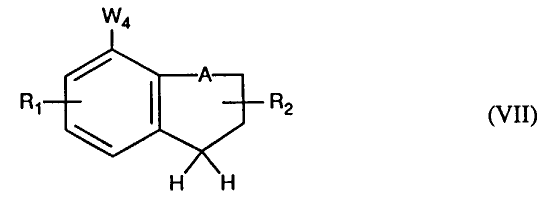 worin W₄ Cyano oder eine Cyanovorstufe wie oben definiert bedeutet, herstellen. Wird eine Cyanovorstufe eingesetzt, so ist diese wiederum nach erfolgter Oxidation in Cyano zu überführen, z.B. wie oben angegeben.

Eine weitere Möglichkeit zur Herstellung der Verbindungen der Formel V, worin die Gruppe CW₁W₂ Carbonyl bedeutet, besteht darin, dass man von Verbindungen der Formel II, worin X Wasserstoff bedeutet, ausgeht und die Cyanogruppe einführt, beispielsweise durch eine Reaktionssequenz analog US-Patent 3,956,363, Beispiel 10, die aus Nitrierung, Reduktion der Nitrogruppe zu Amino, Diazotierung und Umsetzung mit Kupfer(I)cyanid (Sandmeyer-Reaktion) besteht.

Die Herstellung von Aminoguanidinen der Formel III ist an sich bekannt.

Aminoguanidin der Formel III ist an sich bekannt.

### Verfahren (ii):

In den Zwischenprodukten der Formel IV bedeutet W₃ z.B. freies oder funktionell abgewandeltes Carboxy, insbesondere Halogencarbonyl, Cyano, Imino-niederalkoxycarbonyl, Imino-niederalkylthiolcarbonyl oder Thiocarbamoyl.

Die Gruppe W₃ in einer Verbindung der Formel IV kann bei der Herstellung von Amidinen der Formel II z.B. bedeuten: ein Säureadditionssalz eines Imino-niederalkylesters (=̂ Iminoniederalkylether) oder Imino-niederalkylthiolesters, z.B. -C(=NH)-OC₂H₅ · HCl bzw. -C(=NH)-SC₂H₅ · HI, oder Cyano.

Durch die Umsetzung eines Imino-niederalkylesters der Formel IV (als Salz) mit Ammoniak erhält man die unsubstituierten Amidine der Formel I. Cyanoverbindungen der Formel IV können z.B. durch Umsetzung mit einem Alkalimetallamid, z.B. KNH₂, in das Amidin der Formel I überführt werden.

Die Carbonsäureesterimide erhält man z.B. durch säurekatalysierte Anlagerung von Alkoholen an die Nitrile der Formel IV. Aus den Esterimiden erhält man die Amide im Sinne einer Pinner-Spaltung durch thermischen Zerfall der Esterimid-Salze bei Temperaturen oberhalb von etwa 80°C.

Verbindungen der Formel IV, worin W₃ Cyano bedeutet, können z.B. dadurch hergestellt werden, dass man eine Verbindung der Formel V mit einer Verbindung der Formel m gemäss dem Verfahren ii) umsetzt. Aus Verbindungen der Formel IV, worin W₃ Cyano bedeutet, lassen sich die anderen Verbindungen der Formel IV, worin W₃ freies oder anderweitig funktionell abgewandeltes Carboxy bedeutet, in an sich bekannter Weise oder wie oben beschrieben herstellen.

Verbindungen der Formel I können auch durch Umsetzung von Verbindungen der Formel IV, worin W₃ einen Rest -C(=S)-NH₂ bedeutet, unter S-Alkylierung, z.B. mit Triniederalkyloxonium-tetrafluoroborat, und anschliessender Umsetzung mit Ammoniak oder einem entsprechenden Ammoniumsalz, z. B. dem Chlorid, erhalten werden.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren, entweder bereits mit Protonensäuren [PA], oder vorzugsweise mit anderen Protonensäuren, beispielsweise nicht unter die Definition von [PA] fallenden organischen Säuren, wie Ameisensäure, Essigsäure, Methansulfonsäure, oder anorganische Säuren, wie Schwefelsäure, Halogenwasserstoffsäuren, wie HF, HCl, HBr oder HI, ferner Stickstoffwasserstoffsäure oder Phosphorsäure. Besonders bevorzugt sind Halogenwasserstoffsäuren. Die Umsetzung geschieht beispielsweise analog dem Verfahren a) zur Herstellung von Säureadditionssalzen von Basen der Formel I mit Säuren [PA].

Die Säureadditionssalze von Basen der Formel I, insbesondere diejenigen mit anderen Protonensäuren als [PA], können auch in die freien Verbindungen überführt werden. Dies kann z. B. durch Überführung in die freie Base, beispielsweise durch Umsetzung des als Ausgangsmaterial verwendeten Salzes einer Verbindung der Formel I mit einer Hydroxybase, wie einem Alkalihydroxid, z. B. NaOH oder KOH, in wässriger Lösung in An- oder, vorzugsweise, Abwesenheit eines organischen Lösungsmittels, wie unter a) definiert, in die freie Base, ferner durch Dialyse, mittels Ionenaustauschern oder durch Gelchromatographie, geschehen.

Die Herstellung von Ausgangsverbindungen der Formeln II, III, und IV erfolgt wie bei der näheren Beschreibung der Verfahren i) und ii) dargelegt.

Infolge der engen Beziehung zwischen den Verbindungen der Formeln I, II, III, IV und auch V, VI und VII in freier Form und, sofern die genannten Verbindungen salzbildende Gruppen enthalten, in Form von Salzen sind vor- und nachstehend unter den freien Verbindungen bzw. ihren Salzen sinn- und zweckmässig gegebenenfalls auch die entsprechenden Salze, z. B. Säureadditionssalze oder auch Salze mit Basen, bzw. die freien Verbindungen zu verstehen.

Die Herstellung von Salzen, beispielsweise der Verbindungen der Formel II, III und IV mit salzbildenden Gruppen, erfolgt analog der Herstellung von Salzen der Basen der Formel I (Verfahren a).

Die Verbindungen, einschliesslich ihrer Salze, können auch in Form von Hydraten erhalten werden, oder ihre Kristalle können z.B. das zur Kristallisation verwendete Lösungsmittel einschliessen.

Gemäss den Verfahren i) oder ii) erhältliche Gemische von Isomeren können in an sich bekannter Weise in die einzelnen Isomeren aufgetrennt werden.

Die oben angeführten Reaktionen unter den Verfahren i) oder ii) können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. bei Raumtemperatur oder beim Siedepunkt des verwendeten Lösungsmittels im jeweiligen Reaktionsgemisch, unter atmosphärischem Druck oder in einem geschlossenen Gefäss, gegebenenfalls unter Druck, und/oder in einer inerten Atmosphäre, z.B. unter einer Stickstoffatmosphäre.

Nachfolgend ist zu rein illustrativen Zwecken die Herstellung einiger Ausgangsverbindungen beschrieben. Temperaturen werden in Grad Celsius angegeben. Folgende Abkürzungen werden verwendet: abs. = absolut (wasserfrei); D₂O = deuteriertes Wasser; DMF = N,N-Dimethylformamid; DMSO-d₆ = perdeuteriertes Dimethylsulfoxid; Ether = Diethylether; Essigester = Essigsäureethylester; IR = Infrarotspektrospkopie; Smp. = Schmelzpunkt; THF = Tetrahydrofuran; MS (FAB) = Massenspektrum ("Fast Atom Bombardment").

1) **Ausgangsverbindung A:**
**4-Amino-1-indanon-2'-amidinohydrazon-dihydrochlorid**

Eine Lösung von 3,8 g (27,9 mMol) Aminoguanidin-hydrogencarbonat in 200 ml Wasser und 14,7 ml 2N Salzsäure wird auf 60° erwärmt und unter Rühren innerhalb von 30 min mit einer Lösung von 5,85 g (27,8 mMol) 4-Amidino-1-indanon-hydrochlorid in 200 ml Methanol versetzt. Das Reaktionsgemisch wird 24 h am Rückfluss gekocht und nach dem Abkühlen zur Trockne eingedampft. Der Rückstand wird in 50 ml Ethanol suspendiert, filtriert, mit Ethanol und Ether gewaschen und getrocknet. Man erhält so die Titelverbindung, die 1 Mol Kristallwasser enthält, Smp. >330°; MS(FAB): (M+H)⁺=231; ¹H-NMR (D₂O): δ=8,08 (d,1H); 7,75 (d,1H); 7,58 (t,1H); 3,35 (m, 2H); 2,96 (m,2H).

Die Vorstufen werden wie folgt hergestellt:

### (a) 4-Thiocarbamoyl-1-indanon

Eine Lösung von 12,1 g (77 mMol) 4-Cyano-1-indanon [Coll. Czechoslov. Chem. Commun. 43, 3227 (1978)] in 220 ml Pyridin und 10,6 ml (77 mMol) Triethylamin wird bei 40° während 3 h mit Schwefelwasserstoff gesättigt und weitere 16 h bei der gleichen Temperatur gerührt. Das Reaktionsgemisch wird nach dem Abkühlen zur Trockne eingedampft und der Rückstand mit 300 ml Wasser versetzt. Das auskristallisierte gelbe Produkt wird abgesaugt, mit Wasser gewaschen, getrocknet und aus Essigester umkristallisiert. Man erhält so die Titelverbindung, Smp. 197°(Zers.).

### (b) 4-Amidino-1-indanon-hydrochlorid

Eine Lösung von 9,8 g (51,3 mMol) 4-Thiocarbamoyl-1-indanon in 500 ml abs. Methylenchlorid wird bei Raumtemperatur unter Argon mit 10,8 g (54 mMol) Triethyloxonium-tetrafluoroborat versetzt. Nach 16 h gibt man zu der Reaktionslösung ein Gemisch von 4,2 g Kaliumcarbonat und 4,2 ml Wasser. Man rührt kurz nach, filtriert und wäscht das Filtrat mit Wasser. Die organische Phase wird über Magnesiumsulfat getrocknet, filtriert und eingedampft. Der so erhaltene rohe Ethylthio-iminoether wird in 160 ml abs. Ethanol gelöst, mit 3,3 g (60 mMol) Ammoniumchlorid versetzt und 20 h am Rückfluss erhitzt. Nach dem Abkühlen wird das Reaktionsgemisch zur Trockne eingedampft. Die Titelverbindung wird durch Chromatographie an 1000 ml Amberlite® ER-180 Harz (Wasser als Eluiermittel) gereinigt und aus Ethanol/Ether umkristallisiert, Smp. 215-218° (Zers.).

2) **Ausgangsverbindung B:**
**4-Amidino-1-indanon-2'-(N-hydroxyamidino)-hydrazon-dihydrochlorid**

Eine Lösung von 316 mg (1,5 mMol) 4-Amidino-1-indanon-hydrochlorid (siehe Vorstufe 1 b) unter Ausgangsverbindung A) in 7 ml Methanol wird mit einer Lösung von 394 mg (1,5 mMol) 1-Amino-3-hydroxyguanidin-4-toluolsulfonat in 6 ml Wasser und 0,75 ml (1,5 mMol) 2N Salzsäure versetzt, 2 h am Rückfluss erhitzt und 16 h bei Raumtemperatur gerührt. Das Reaktionsgemisch wird eingedampft und der Rückstand durch Chromatographie (Pharmacia-Säule SR-28-100) an Kieselgel OPTI-UP C₁₂® (Wasser als Eluiermittel, 5 ml-Fraktionen, Durchflussgeschwindigkeit 27,5 ml/h) gereinigt. Der Inhalt der Fraktionen 58-78 wird vereinigt, eingedampft und der Rückstand aus Ethanol kristallisiert. Man erhält so die Titelverbindung in Form von wachsartigen Kristallen, MS (FAB): (M+H)⁺ = 247; ¹H-NMR (D₂O):δ=8,06 (d,1H); 7,73 (d,1H); 7,58 (t,1H); 3,36 (m, 2H); 2,98 (m,2H).

3) **Ausgangsverbindung D:**
**4-Thiocarbamoyl-1-indanon-2'-amidinohydrazon-hydrochlorid**

Eine Lösung von 1,9 g (10 mMol) 4-Thiocarbamoyl-1-indanon (hergestellt wie unter Augangsverbindung A, 1 a) beschrieben) in 50 ml Ethanol wird mit 1,36 g (10 mMol) Aminoguanidin-hydrogencarbonat und 10 ml 2N Salzsäure versetzt und 24 h am Rückfluss erhitzt. Das Reaktionsgemisch wird nach dem Abkühlen zur Trockne eingedampft, wobei man die Titelverbindung erhält.

4) **Ausgangsverbindung E:**
**4-(N-Hydroxyamidino)-1-indanon-2'-amidinohydrazon-dihydrochlorid**

0,2 g (3 mMol) Hydroxylamin-hydrochlorid werden in 1 ml abs. Ethanol suspendiert und mit 2 ml einer 1N Natrium-ethoxid-Lösung in Ethanol versetzt. Dieses Gemisch wird 1 h gerührt und filtriert. Zum Filtrat wird eine Lösung von 0,26 g (1 mMol) 4-Cyano-1-indanon-2'-amidinohydrazon-hydrochlorid (siehe unten unter 18 a)) in 2 ml Wasser gegeben, und man erhitzt 6 h am Rückfluss. Nach dem Abkühlen wird das Reaktionsgemisch eingedampft und die Titelverbindung aus Wasser kristallisiert, Smp. >250°; ¹H-NMR (DMSO-d₆ + D₂O): δ = 8,12 (m,1H); 7,55 (m,2H); 3,22 (m,2H); 2,83 (m,2H).

Weitere Verfahren zur Herstellung von Ausgangsverbindung A)
(4-Amidino-1-indanon-2'-amidinohydrazon-dihydrochlorid):

5) Analog dem für Ausgangsverbindung A unter 1 b) beschriebenen Verfahren wird 4-Thiocarbamoyl-1-indanon-2'-amidinohydrazon-hydrochlorid (Ausgangsverbindung D) mit Triethyloxonium-tetrafluoroborat und Ammoniumchlorid umgesetzt, wobei man Ausgangsverbindung A erhält, Smp. >330°; MS (FAB): (M+H)⁺ = 231; ¹1H-NMR (D₂O): δ=8,08 (d,1H); 7,75 (d,1H); 7,58 (t,1H); 3,35 (m,2H); 2,96 (m,2H).

6) Eine Lösung von 0,26 g (1 mMol) 4-Cyano-1-indanon-2'-amidinohydrazon-hydrochlorid in 5 ml abs. Methanol wird mit 1,2 ml einer 1N Natriummethoxid-Lösung in Methanol versetzt und 16 h am Rückfluss erhitzt. Nach dem Abkühlen gibt man zum Reaktionsgemisch 0,16 g (3 mMol) festes Ammoniumchlorid zu und rührt 1 h bei 60°. Das Reaktionsgemisch wird danach eingedampft und der Rückstand aus verdünntem Ethanol kristallisiert . Man erhält so Ausgangsverbindung A, Smp. >330°.

Die Vorstufe wird wie folgt hergestellt:

### (a) 4-Cyano-1-indanon-2'-amidinohydrazon-hydrochlorid

Analog dem für Ausgangsverbindung A unter 1) genannten Verfahren werden 314 mg (2 mMol) 4-Cyano-1-indanon in 20 ml Methanol gelöst, mit einer Lösung von 272 mg (2 mMol) Aminoguanidin-hydrogencarbonat in 9 ml Wasser und 1 ml 2N Salzsäure versetzt und 4 Tage am Rückfluss gerührt. Nach dem Abkühlen wird das Reaktionsgemisch zur Trockne eingedampft und der Rückstand aus Wasser kristallisiert. Man erhält so die Titelverbindung, Smp. >230°; ¹H-NMR (DMSO-d₆/D₂O): δ=8,16 (d,1H); 7,9 (d,1H); 7,55 (t,1H); 3,28 (m,2H); 2,9 (m,2H); IR(Nujol (Paraffin)): 2190 cm⁻¹ (CN).

7) Eine Lösung von 6,12 g (45 mMol) Aminoguanidin-hydrogencarbonat in 100 ml Wasser und 46 ml 1N Salzsäure wird mit 9,45 g (44,9 mMol) 4-Amidino-1-indanon-hydrochlorid (siehe unter Ausgangsverbindung A, 1 b)) versetzt und 24 h bei 24° gerührt. Das auskristallisierte Produkt wird abgesaugt, mit wenig Wasser gewaschen und aus 300 ml Wasser umkristallisiert. Man erhält so Ausgangsverbindung A, die 1 Mol Kristallwasser enthält, Smp. >330°; MS (FAB): (M + H)⁺ = 231; ¹H-NMR (D₂O): δ = 8,08 (d,1H); 7,75 (d,1H); 7,58 (t,1H); 3,35 (m,2H); 2,96 (m,2H).

8) Ein Gemisch von 0,32 g (1 mMol) 4-(N-Hydroxyamidino)-1-indanon-2'-amidinohydrazon-dihydrochlorid (Ausgangsverbindung E), 0,36 ml (2 mMol) Triethylamin und 0,2 g (1 mMol) Eisenpentacarbonyl in 10 ml abs. THF wird 16 h am Rückfluss gekocht. Das Reaktionsgemisch wird danach eingedampft und der Rückstand aus verdünnter Salzsäure kristallisiert, wobei man Ausgangsverbindung A erhält, Smp. >330°.

### Beispiele

Die folgenden Beispiele dienen zur Illustration der Erfindung, schränken deren Umfang jedoch in keiner Weise ein.

Temperaturen werden in Celsiusgraden (°C) angegeben. Erfolgt keine Temperaturangabe, so wird die jeweilige Reaktion bei Raumtemperatur durchgeführt. Falls eingedampft wird, geschieht dies mit einem Rotationsverdampfer, sofern nichts anderes angegeben ist.

Die Werte für Protonen-Kernresonanzspektroskopie werden in ppm ("Parts Per Million") bezogen auf Tetramethylsilan (δ = 0) als internen Standard angegeben. d = Duplett, s = Singulett, t = Triplett, m = Multiplett.

Bei Elementaranalysen sind die Summenformel, das Molekulargewicht, berechnete und gefundene Analysewerte angegeben.

Die verwendeten Kurzbezeichnungen und Abkürzungen haben folgende Bedeutungen:

| | |
|---|---|
| Anal. | Elementaranalyse |
| ber. | berechnet |
| gef. | gefunden |
| D₂O | Dideuteriumoxid |
| DMSO-d₆ | vollständig deuteriertes Dimethylsulfoxid |
| ¹H-NMR | Protonmagnetische Kernresonanzspektroskopie |
| Smp. | Schmelzpunkt |
| Zers. | unter Zersetzung |

### Beispiel 1: 4-Amidino-1-indanon-2-amidinohydrazon-dicyclamat

Eine Lösung von 460 mg (2 mMol) 4-Amidinoindanon-1-amidinohydrazon in 80 ml Methanol wird mit einer Lösung von 717 mg (4 mMol) N-Cyclohexylsulfaminsäure in 20 ml Methanol versetzt und zur Trockne eingedampft. Der Rückstand wird in Ethanol gelöst und durch Zugabe von Diethylether kristallisiert. Man erhält so die Titelverbindung, Smp. 210° (Zers.); ¹H-NMR (D₂O): δ 7,97 (d,1H); 7,64 (d,1H); 7,47 (t, 1H); 3,25 (m,2H); 2,9 (m,4H); 1-2 (m,20H); Anal. für C₂₃H₄₀N₈O₆S₂ (588.75): ber. 46.92% C, 6,85% H, 19,03% N, gef. 46,5% C, 6,9% H, 19,0% N.

Das Ausgangsmaterial wird folgendermassen hergestellt:

### a) 4-Amidino-1-indanon-2'-amidinohydrazon

9,63 g (30 mMol) 4-Amidino-1-indanon-2'-amidinohydrazondihydrochlorid (Ausgangsverbindung A, hergestellt nach einem der unter "Ausgangsverbindungen" beschriebenen Verfahren, z. B. nach 16)) werden in 900 ml auf 70-80 °C erwärmtem destilliertem Wasser gelöst und auf 10 °C gekühlt. Diese Lösung wird dann tropfenweise unter Rühren mit 30 ml 2N Natronlauge versetzt. Das ausgeschiedene Produkt wird abgesaugt, mit wenig Eiswasser gewaschen und getrocknet. Es stellt die Titelverbindung dar, Smp. 250° (Zers.).

### Beispiel 2:

### 4-Amidino-1-indanon-2'-amidinohydrazon-dioctanoat

Eine Lösung von 460 mg (2 mMol) 4-Amidinoindanon-1-amidinohydrazon (Beispiel 1 a)) in 80 ml Methanol wird mit 631 µl (4 mMol) Octansäure versetzt und zur Trockne eingedampft. Der Rückstand wird in Ethanol gelöst und durch Zugabe von Diethylether kristallisiert. Man erhält so die Titelverbindung, Smp. 190° (Zers.); ¹H-NMR (DMSO-d₆): δ 8,03 (d,1H); 7,52 (d,1H); 7,47 (t,1H); 3,19 (m,2H); 2,85 (m,2H); 1,98 (t,4H); 1,42 (m,4H); 1,19 (s,24H); 0,81 (t,6H); Anal. für C₂₇H₄₆N₆O₄ (518,70): ber. 62,52% C, 8,94% H, 16,20% N, gef. 62,3% C, 8,9% H, 16,2% N.

### Beispiel 3: 4-Amidino-1-indanon-2'-amidinohydrazon-disalicylat

Eine Lösung von 690 mg (3 mMol) 4-Amidinoindanon-1-amidinohydrazon (Beispiel 1 a)) in 100 ml Methanol wird mit einer Lösung von 830 mg (6 mMol) Salicylsäure in 50 ml Ethanol versetzt und das Reaktionsgemisch wird auf die Hälfte eingedampft. Diese Lösung wird mit 70 ml Wasser versetzt, wobei die Titelverbindung auskristallisiert, Smp. 206-9° (Zers.); Anal. für C₂₅H₂₆N₆O₆ (507.1): ber. 59,13% C, 5,19% H, 16,55% N, gef. 59,3% C, 5,2% H, 16,8% N.

### Beispiel 4: 4-Amidino-1-indanon-2'-amidinohydrazon-dibenzolsulfonat

Eine Lösung von 690 mg (3 mMol) 4-Amidinoindanon-1-amidinohydrazon (Beispiel 1 a)) in 100 ml Methanol wird mit einer Lösung von 975 mg (3 mMol) Benzolsulfonsäure in 50 ml Methanol versetzt und zur Trockne eingedampft. Der Rückstand wird aus Ethanol kristallisiert. Man erhält so die Titelverbindung, Smp. >250° (Zers.); ¹H-NMR (D₂O): δ 7,38-7,95 (m,16H); 3,21 (m,2H); 2,78 (m,2H); Anal. für C₂₃H₂₆N₆O₆S₂ (546,63): ber 50,54% C, 4,79% H, 15,37% N, gef. 50,4% C, 4,8% H, 15,6% N.

### Beispiel 5: weitere Salze

### Beispiel 5a: 4-Amidino-1-indanon-2'-amidinohydrazon-succinat

Eine Lösung von 920 mg (4 mMol) 4-Amidino-1-indanon-2'-amidinohydrazon in 120 ml Methanol wird mit einer Lösung von 472 mg (4 mMol) Bernsteinsäure in 120 ml Methanol versetzt. Das auskristallisierte Produkt wird abgesaugt, mit wenig Methanol gewaschen und getrocknet und stellt die Titelverbindung dar, Smp. 200° (Zers.); Anal. für C₁₅H₂₀N₆O₄·1,04 H₂O (367,10): ber. 49,08% C, 6,06% H, 22,89% N, gef. 49,13% C, 6,07% H, 23,04% N.

### Beispiel 5b: 4-Amidino-1-indanon-2'-amidinohydrazon-adipat

Eine Lösung von 230 mg (1 mMol) 4-Amidino-1-indanon-2'-amidinohydrazon in 30 ml Methanol wird mit einer Lösung von 146 mg (1 mMol) Adipinsäure in 25 ml Ethanol versetzt. Das auskristallisierte Produkt wird abgesaugt, mit wenig Ethanol gewaschen und getrocknet und stellt die Titelverbindung dar, Smp. 200° (Zers.); Anal. für C₁₇H₂₄N₆O₄·0,25 H₂O (380,92): ber. 53,60% C, 6,48% H, 22,06% N, gef. 53,79% C, 6,73% H, 21,93% N.

### Beispiel 5c: 4-Amidino-1-indanon-2'-amidinohydrazon-1,5-naphthalindisulfonat

Eine Lösung von 920 mg (4 mMol) 4-Amidino-1-indanon-2'-amidinohydrazon in 120 ml Methanol wird mit einer Lösung von 1,49 g (4 mMol) 1,5-Naphthalindisulfonsäure in 100 ml Methanol versetzt. Das auskristallisierte Produkt wird abgesaugt, mit wenig Methanol gewaschen und getrocknet und stellt die Titelverbindung dar, Smp. >250°; Anal. für C₂₁H₂₂N₆O₆S₂·2,36 H₂O (561,09): ber. 44,95% C, 4,80% H, 14,98% N, gef. 45,06% C, 4,98% H, 15,21% N.

### Beispiel 5d: 4-Amidino-1-indanon-2'-amidinohydrazon-ethandisulfonat

Analog einem der vor- und nachstehend genannten Beispiele wird 4-Amidino-1-indanon-2'-amidino-hydrazon mit Hilfe von 1,2-Ethandisulfonsäure in die Titelverbindung überführt.

### Beispiel 5e: 4-Amidino-1-indanon-2'-amidinohydrazon-L-tartrat

Eine Lösung von 920 mg (4 mMol) 4-Amidino-1-indanon-2'-amidinohydrazon in 120 ml Methanol wird mit einer Lösung von 600 mg (4 mMol) L-(+)-Weinsäure in 100 ml Methanol versetzt. Das auskristallisierte Produkt wird abgesaugt, mit wenig Methanol gewaschen und getrocknet und stellt die Titelverbindung dar, Smp. 190° (Zers.); Anal. für C₁₅H₂₀N₆O₆·0,26 H₂O (385,04): ber. 46,79% C, 5,37% H, 21,83% N, gef. 46,83% C, 5,43% H, 21,87% N.

### Beispiel 5f: 4-Amidino-1-indanon-2'-amidinohydrazon-zitrat

Eine Lösung von 230 mg (1 mMol) 4-Amidino-1-indanon-2'-amidinohydrazon in 30 ml Methanol wird mit einer Lösung von 210 mg (1 mMol) Zitronensäure in 10 ml Methanol versetzt. Das auskristallisierte Produkt wird abgesaugt, mit wenig Methanol gewaschen und getrocknet und stellt die Titelverbindung dar, Smp. >220° (Zers.); Anal. für C₁₇H₂₂N₆O₇ (422,40): ber. 48,34% C, 5,25% H, 19,90% N, gef. 48,23 %C, 5,33% H, 20,07% N.

### Beispiel 5g: 4-Amidino-1-indanon-2'-amidinohydrazon-dilactat

Analog einem der vor- und nachstehend genannten Beispiele wird 4-Amidino-1-indanon-2'-amidinohydrazon mit Hilfe von Milchsäure in die Titelverbindung überführt.

### Beispiel 6:

Kapseln enthaltend 0,25 g Wirkstoff, z.B. eines der Säureadditionssalze der Beispiele 1-5, können wie folgt hergestellt werden:

| Zusammensetzung (für 5000 Kapseln) | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

### Beispiel 7:

Es werden 10000 Tabletten hergestellt, die je 5 mg Wirkstoff, z.B. eines der in den Beispielen 1-5 hergestellten Säureadditionssalze, enthalten:

| Zusammensetzung: | |
|---|---|
| Wirkstoff | 50,00 g |
| Milchzucker | 2535,00 g |
| Maisstärke | 125,00 g |
| Polyethylenglykol 6.000 | 150,00 g |
| Magnesiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

## Patentansprüche

1. Ein Säureadditionssalz einer Base der Formel I 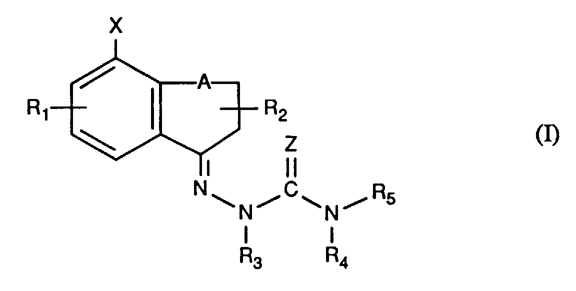 worin
A eine direkte Bindung bedeutet,
X für einen Rest -C(=NH)-NH₂ steht;
Z für NH steht; und
R₁, R₂, R₃, R₄ und R₅ jeweils Wasserstoff bedeuten;
mit einer Säure [PA], welche eine ein- oder mehrprotonige Säure ausgewählt aus Kohlensäure, Alkansäuren, die unsubstituiert oder ein bis mehrfach substituiert sind, ausser Ameisensäure, unsubstituierter Essigsäure, Lysin und Arginin, Alkensäuren, die unsubstituiert oder substituiert sind, ausser unsubstituierter Fumarsäure, Cycloalkylcarbonsäuren, Arylcarbonsäuren, Arylniederalkylcarbonsäuren, worin Niederalkyl Methyl oder Ethyl bedeutet und unsubstituiert oder substituiert ist, Aryl-C₂-C₄-alkenylcarbonsäuren, Heterocyclylcarbonsäuren, Alkansulfonsäuren, die unsubstituiert oder substituiert sind, ausser unsubstituierter Methansulfonsäure, aromatische Sulfonsäuren, Alkylschwefelsäuren, N-substituierten Sulfaminsäuren, organischen Säuren ohne Carboxy-, Sulfo-, Sulfat- oder Phosphogruppen, und aus Pyrophosphorsäure und Iodwasserstoff, bedeutet, oder Tautomere davon.

2. Ein Säureadditionssalz gemäss Anspruch 1 von einer Base der Formel I mit einer Säure [PA], welche Kohlensäure, Propionsäure, Octansäure, Decansäure, Dodecansäure, Glykolsäure, Milchsäure, 2-Hydroxybuttersäure, Gluconsäure, Glucosemonocarbonsäure, Bernsteinsäure, Adipinsäure, Pimelinsäure, Suberinsäure, Azelainsäure, Äpfelsäure, Weinsäure, Zitronensäure, Glucarsäure, Galactarsäure, Glutaminsäure, Asparaginsäure, N-Methylglycin, Acetylaminoessigsäure, N-Acetylasparagin, N-Acetylcystin, Brenztraubensäure, Acetessigsäure, Phosphoserin, 2- oder 3-Glycerophosphorsäure, Glucose-6-phosphorsäure, Glucose-1phosphorsäure, Fructose1,6-bisphosphorsäure, Maleinsäure, Hydroxymaleinsäure, Methylmaleinsäure, Cyclohexancarbonsäure, Adamantancarbonsäure, Benzoesäure, Salicylsäure, 1- oder 3-Hydroxynaphthyl-2-carbonsäure, 3,4,5-Trimethoxybenzoesäure, 2-Phenoxybenzoesäure, 2-Acetoxybenzoesäure, 4-Aminosalicylsäure, Phthalsäure, Phenylessigsäure, Mandelsäure, Zimtsäure, Nicotinsäure, Isonicotinsäure, Glucuronsäure, Galacturonsäure, Ethansulfonsäure, 2-Hydroxyethansulfonsäure, Ethan-1,2-disulfonsäure, Benzolsulfonsäure, 2-Naphthalinsulfonsäure, 1,5-Naphthalindisulfonsäure, 2-, 3- oder 4-Methylbenzolsulfonsäure, Methylschwefelsäure, Ethylschwefelsäure, Dodecylschwefelsäure, N-Cyclohexylsulfaminsäure, N-Methyl-, N-Ethyl- oder N-Propylsulfaminsäure, oder Ascorbinsäure bedeutet, oder Tautomere davon.

3. Ein Säureadditionssalz gemäss Anspruch 1 einer Base der Formel I mit einer Säure [PA], welche N-Cyclohexylsulfaminsäure, Octansäure, Salicylsäure oder Benzolsulfonsäure bedeutet.

4. Ein Säureadditionssalz gemäss Anspruch 1 einer Base der Formel I mit einer Säure [PA], welche aus Zitronensäure, Milchsäure und Weinsäure ausgewählt ist.

5. Ein Säureadditionssalz gemäss Anspruch 4 einer Base der Formel I mit Milchsäure.

6. Ein Säureadditionssalz gemäss Anspruch 1 einer Base der Formel I mit einer Säure [PA], welche aus Bernsteinsäure, Adipinsäure und 1,5-Naphthalindisulfonsäure ausgewählt ist.

7. Ein Säureadditionssalz gemäss Anspruch 1 einer Base der Formel I mit einer Säure [PA], welche Bernsteinsäure, Adipinsäure, Ethandisulfonsäure oder 1,5-Naphthalindisulfonsäure bedeutet.

8. Das Säureadditionssalz gemäss Anspruch 1 einer Base der Formel I mit der Säure [PA], welche aus Adipinsäure und Benzolsulfonsäure ausgewählt ist.

9. Pharmazeutische Präparate enthaltend ein Säureadditionssalz gemäss einem der Ansprüche 1 bis 8 und mindestens ein pharmazeutisch verwendbares Trägermaterial.

10. Ein Säureadditionssalz gemäss einem der Ansprüche 1 bis 8 zur Anwendung in einem Verfahren zur therapeutischen Behandlung des tierischen oder menschlichen Körpers.

11. Ein Säureadditionssalz gemäss einem der Ansprüche 1 bis 8 zur Verwendung bei der Behandlung von Krankheiten, die auf eine Hemmung des Enzyms S-Adenosylmethionindecarboxylase ansprechen.

12. Verwendung eines Säureadditionssalzes gemäss einem der Anprüche 1 bis 8 zur Herstellung pharmazeutischer Präparate für die Behandlung von Krankheiten, die auf eine Hemmung des Enzyms S-Adenosylmethionindecarboxylase ansprechen.

13. Verfahren zur Herstellung eine Säureadditionssalzes gemäss Anspruch 1, dadurch gekennzeichnet, dass man
a) eine Base der Formel I 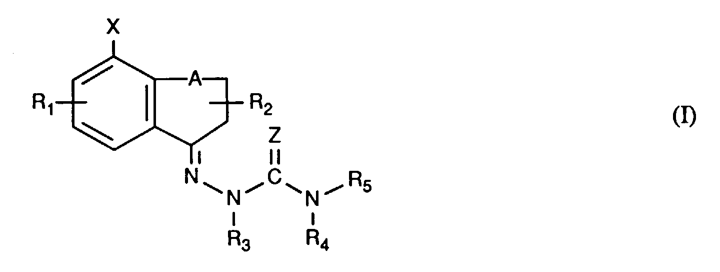 worin die Reste die für Säureadditionssalze von Basen der Formel I in Anspruch 1 genannten Bedeutungen haben, mit einer Säure [PA], wobei [PA] die in Anspruch 1 genannten Bedeutungen hat, umsetzt, oder
b) eine Verbindung der Formel 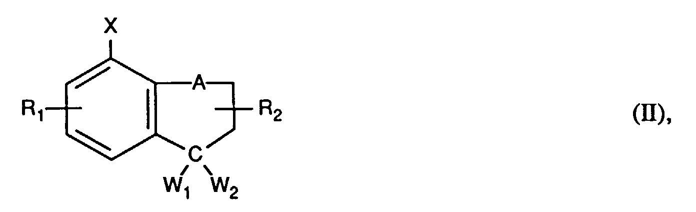 worin die Gruppe CW₁W₂ für Carbonyl, funktionell abgewandeltes Carbonyl oder geschütztes Carbonyl steht und A, X, R₁ und R₂ die für Säureadditionssalze der Formel I in Anspruch 1 genannten Bedeutungen haben, mit einem Amin der Formel 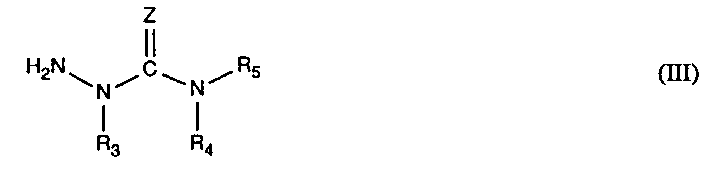 worin Z, R₃, R₄ und R₅ die für Säureadditionssalze von Basen der Formel I in Anspruch 1 genannten Bedeutungen haben, in Gegenwart einer Säure [PA] mit den in Anspruch 1 genannten Bedeutungen kondensiert, oder
c) in einer Verbindung der Formel 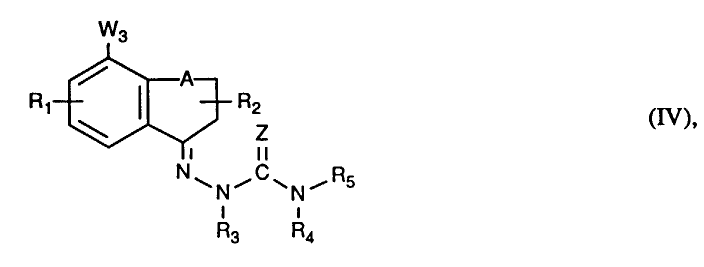 worin W₃ einen Rest, der in eine Gruppe X in einer Base der Formel I gemäss Anspruch 1 überführt werden kann, bedeutet und A, Z, R₁, R₂, R₃, R₄ und R₅ wie unter Formel I in Anspruch 1 definiert sind, den Rest W₃ in Gegenwart einer Säure [PA] mit den in Anspruch 1 genannten Bedeutungen in die Gruppe X überführt; oder
d) ein beliebiges Säureadditionssalz von einer Base der Formel I mit einer Säure, die nicht unter die Definition von [PA] in Anspruch 1 fällt, in ein Säureadditionssalz von einer Base der Formel I mit einer Säure [PA] mit den in Anspruch 1 genannten Bedeutungen umwandelt;
und, wenn gewünscht, ein erhaltenes Säureadditionssalz einer Base der Formel I mit einer Säure [PA] in ein anderes Säureadditionssalz einer Base der Formel I mit einer Säure [PA] umwandelt, und/oder, wenn gewünscht, Isomerengemische auftrennt in die einzelnen Isomeren.

## Claims

1. An acid addition salt of a base of formula I 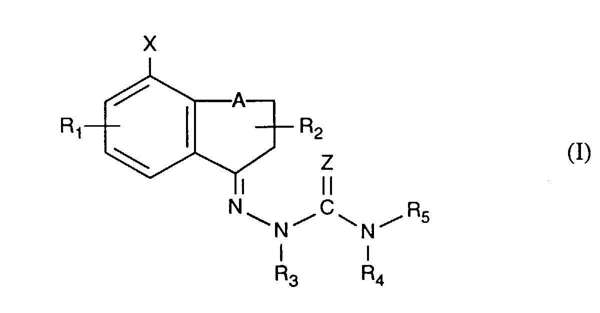 wherein
A is a direct bond,
X is a radical -C(=NH)-NH₂;
Z is NH; and
R₁, R₂, R₃, R₄ and R₅ are each hydrogen;
with an acid [PA] that is a mono- or poly-protonic acid selected from carbonic acid, alkanoic acids that are unsubstituted or mono- or poly-substituted, with the exception of formic acid, unsubstituted acetic acid, lysine and arginine; alkenoic acids that are unsubstituted or substituted, with the exception of unsubstituted fumaric acid; cycloalkylcarboxylic acids, arylcarboxylic acids, aryl-lower alkylcarboxylic acids, wherein lower alkyl is methyl or ethyl and is unsubstituted or substituted, aryl-C₂-C₄alkenylcarboxylic acids, heterocyclylcarboxylic acids, alkanesulfonic acids that are unsubstituted or substituted, with the exception of unsubstituted methanesulfonic acid; aromatic sulfonic acids, alkylsulfuric acids, N-substituted sulfamic acids, organic acids without carboxy, sulfo, sulfate or phospho groups, and pyrophosphoric acid and hydriodic acid, or a tautomer thereof.

2. An acid addition salt according to claim 1 of a base of formula I with an acid [PA] that is carbonic acid, propionic acid, octanoic acid, decanoic acid, dodecanoic acid, glycolic acid, lactic acid, 2-hydroxybutyric acid, gluconic acid, glucose monocarboxylic acid, succinic acid, adipic acid, pimelic acid, suberic acid, azelaic acid, malic acid, tartaric acid, citric acid, glucaric acid, galactaric acid, glutamic acid, aspartic acid, N-methylglycine, acetylaminoacetic acid, N-acetylasparagine, N-acetylcystine, pyruvic acid, acetoacetic acid, phosphoserine, 2- or 3-glycerophosphoric acid, glucose-6-phosphoric acid, glucose-1-phosphoric acid, fructose-1,6-bisphosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, mandelic acid, cinnamic acid, nicotinic acid, isonicotinic acid, glucuronic acid, galacturonic acid, ethanesulfonic acid, 2-hydroxyethanesulfonic acid, ethane-1,2-disulfonic acid, benzenesulfonic acid, 2-naphthalenesulfonic acid, 1,5-naphthalenedisulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propylsulfamic acid, or ascorbic acid, or a tautomer thereof.

3. An acid addition salt according to claim 1 of a base of formula I with an acid [PA] that is N-cyclohexylsulfamic acid, octanoic acid, salicylic acid or benzenesulfonic acid.

4. An acid addition salt according to claim 1 of a base of formula I with an acid [PA] that is selected from citric acid, lactic acid and tartaric acid.

5. An acid addition salt according to claim 4 of a base of formula I with lactic acid.

6. An acid addition salt according to claim 1 of a base of formula I with an acid [PA] that is selected from succinic acid, adipic acid and 1,5-naphthalenedisulfonic acid.

7. An acid addition salt according to claim 1 of a base of formula I with an acid [PA] that is succinic acid, adipic acid, ethanedisulfonic acid or 1,5-naphthalenedisulfonic acid.

8. The acid addition salt according to claim 1 of a base of formula I with the acid [PA] that is selected from adipic acid and benzenesulfonic acid.

9. A pharmaceutical composition comprising an acid addition salt according to any one of claims 1 to 8 and at least one pharmaceutically acceptable carrier.

10. An acid addition salt according to any one of claims 1 to 8 for use in a method for the therapeutic treatment of the animal or human body.

11. An acid addition salt according to any one of claims 1 to 8 for use in the treatment of diseases that respond to inhibition of the enzyme S-adenosylmethionine decarboxylase.

12. The use of an acid addition salt according to any one of claims 1 to 8 for the preparation of a pharmaceutical composition for the treatment of diseases that respond to inhibition of the enzyme S-adenosylmethionine decarboxylase.

13. A process for the preparation of an acid addition salt according to claim 1, wherein
a) a base of formula I 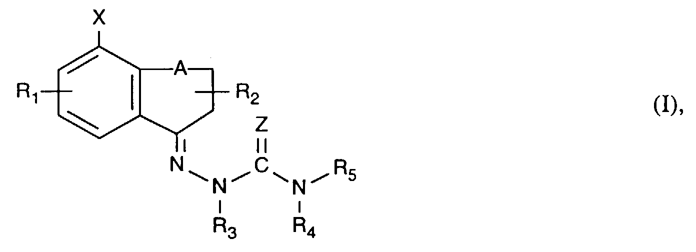 wherein the radicals are as defined for acid addition salts of bases of formula I in claim 1, is reacted with an acid [PA], [PA] being as defined in claim 1, or
b) a compound of the formula 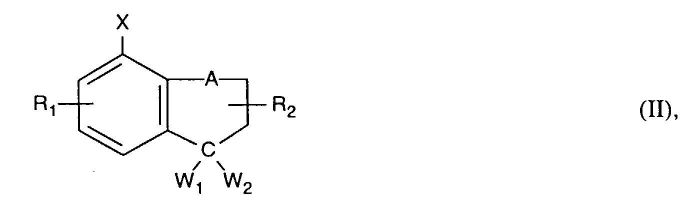 wherein the group CW₁W₂ is carbonyl, functionally modified carbonyl or protected carbonyl and A, X, R₁ and R₂ are as defined for acid addition salts of formula I in claim 1, is condensed with an amine of the formula 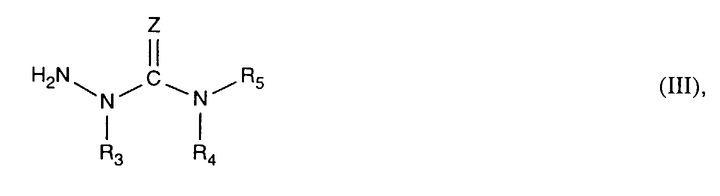 wherein Z, R₃, R₄ and R₅ are as defined for acid addition salts of bases of formula I in claim 1, in the presence of an acid [PA] that is as defined in claim 1, or
c) in a compound of the formula 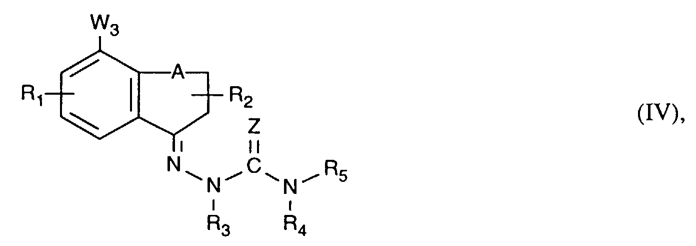 wherein W₃ is a radical that can be converted into a group X in a base of formula I according to claim 1 and A, Z, R₁, R₂, R₃, R₄ and R₅ are as defined under formula I in claim 1, the radical W₃ is converted into the group X in the presence of an acid [PA] that is as defined in claim 1; or
d) any desired acid addition salt of a base of formula I with an acid that does not fall within the definition of [PA] in claim 1 is converted into an acid addition salt of a base of formula I with an acid [PA] that is as defined in claim 1;
and, if desired, a resulting acid addition salt of a base of formula I with an acid [PA] is converted into a different acid addition salt of a base of formula I with an acid [PA], and/or, if desired, isomeric mixtures are separated into the individual isomers.

## Revendications

1. Un sel d'addition d'acides d'une base de formule I 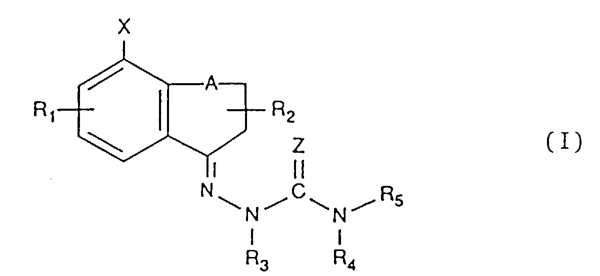 où
A représente une liaison directe;
X un reste -C(=NH)-NH₂;
Z représente NH; et
R₁, R₂, R₃, R₄ et R₅ l'hydrogène;
avec un acide [PA], représentant un acide mono- ou polyprotonique choisi parmi l'acide carbonique, les acides alcanoïques non substitués ou mono-, di- ou polysubstitués, à l'exclusion de l'acide formique, de l'acide acétique non substitué, de la lysine et de l'arginine, les acides alcénoïques non substitués ou substitués, à l'exclusion de l'acide fumarique non substitué, les acides cycloalkylcarboxyliques, les acides arylcarboxyliques, les acides arylalkyle inférieur carboxyliques, l'alkyle inférieur signifiant le méthyle ou l'éthyle et étant non substitué ou substitué, les acides arylalcényle en C₂-C₄ carboxyliques, les acides hétérocyclylcarboxyliques, les acides alcanesulfoniques non substitués ou substitués, à l'exclusion de l'acide méthanesulfonique non substitué, les acides sulfoniques aromatiques, les acides alkylsulfuriques, les acides sulfamiques N-substitués, les acides organiques sans groupes carboxy, sulfo, sulfate ou phospho, l'acide pyrophosphorique et l'acide iodhydrique ou ses tautomères.

2. Un sel d'addition d'acides selon la revendication 1 d'une base de formule I avec un acide [PA] représentant l'acide carbonique, l'acide propionique, l'acide octanoïque, l'acide décanoïque, l'acide dodécanoïque, l'acide glycolique, l'acide lactique, l'acide 2-hydroxybutyrique, l'acide gluconique, l'acide glucosemonocarboxylique, l'acide succinique, l'acide adipique, l'acide pimélique, l'acide subérique, l'acide azélaïque, l'acide malique, l'acide tartrique, l'acide citrique, l'acide glucarique, l'acide galactarique, l'acice glutamique, l'acide aspartique, la N-méthylglycine, l'acide acétylaminoacétique, la N-acétylasparagine, la N-acétylcystine, l'acide pyruvique, l'acide acétacétique, la phosphosérine, l'acide 2- ou 3-glycérophosphorique, l'acide glucose-6-phosphorique, l'acide glucose-1-phosphorique, l'acide fructose-1,6-bisphosphorique, l'acide maléique, l'acide hydroxymaléique, l'acide méthylmaléique, l'acide cyclohexane-carboxylique, l'acide adamantanecarboxylique, l'acide benzoïque, l'acide salicylique, l'acide 1- ou 3-hydroxynaphtyl-2-carboxylique, l'acide 3,4,5-triméthoxybenzoïque, l'acide 2-phénoxybenzoïque, l'acide 2-acétoxybenzoïque, l'acide 4-aminosalicylique, l'acide phtalique, l'acide phénylacétique, l'acide mandélique, l'acide cinnamique, l'acide nicotinique, l'acide isonicotinique, l'acide glucuronique, l'acide galacturonique, l'acide éthanesulfonique, l'acide 2-hydroxyéthanesulfonique, l'acide éthane-1,2-disulfonique, l'acide benzènesulfonique, l'acide 2-naphtalènesulfonique, l'acide 1,5-naphtalènedisulfonique, l'acide 2-, 3- ou 4-méthylbenzènesulfonique, l'acide méthylsulfurique, l'acide éthylsulfurique, l'acide dodécylsulfurique, l'acide N-cyclohexylsulfamique, les acides N-méthyl, N-éthyl- ou N-propylsulfamiques ou l'acide ascorbique ou ses tautomères.

3. Un sel d'addition d'acides selon la revendication 1 d'une base de formule I avec un acide [PA] représentant l'acide cyclohexylsulfamique, l'acide octanoïque, l'acide salicylique ou l'acide benzènesulfonique.

4. Un sel d'addition d'acides selon la revendication 1 d'une base de formule I avec un acide [PA] choisi parmi l'acide citrique, l'acide lactique et. l'acide tartrique.

5. Un sel d'addition d'acides selon la revendication 4 d'une base de formule I avec l'acide lactique.

6. Un sel d'addition d'acides selon la revendication 1 d'une base de formule I avec un acide [PA] choisi parmi l'acide succinique, l'acide adipique et. l'acide l,5-naphtalènedisulfonique.

7. Un sel d'addition d'acides selon la revendication 1 d'une base de formule I avec un acide [PA] représentant l'acide succinique, l'acide adipique, l'acide éthanedisulfonique ou l'acide 1,5-naphtalènedisulfonique.

8. Le sel d'addition d'acides selon la revendication 1 d'une base de formule I avec l'acide [PA] choisi parmi l'acide adipique et l'acide benzènesulfonique.

9. Préparations pharmaceutiques contenant un sel d'addition d'acides selon l'une des revendications 1 à 8 et au moins un support pharmaceutiquement utilisable.

10. Un sel d'addition d'acides selon l'une des revendications 1 à 8 destiné à être utilisé dans un procédé pour le traitement thérapeutique du corps animal ou humain.

11. Un sel d'addition d'acides selon l'une des revendications 1 à 8 destiné à être utilisé pour le traitement des maladies demandant une inhibition de l'enzyme S-adénosylméthioninedécarboxylase.

12. Utilisation d'un sel d'addition d'acides selon l'une des revendications 1 à 8 pour la fabrication de préparations pharmaceutiques destinées au traitement des maladies demandant une inhibition de l'enzyme S-adénosylméthioninedécarboxylase.

13. Procédé pour la préparation d'un sel d'addition d'acides selon la revendication 1, caractérisé
a) en ce que l'on fait réagir une base de formule I 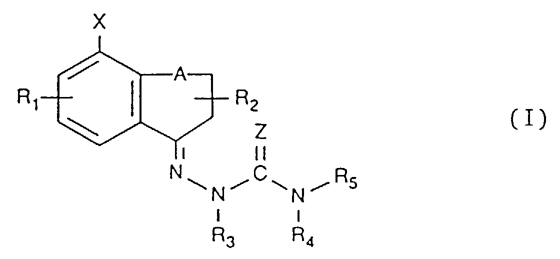 où les restes ont les significations indiquées dans la revendication 1 pour les sels d'addition d'acides des bases de formule I, avec un acide [PA], [PA] ayant les significations indiquées dans la revendication 1, ou
b) en ce que l'on condense un composé de formule 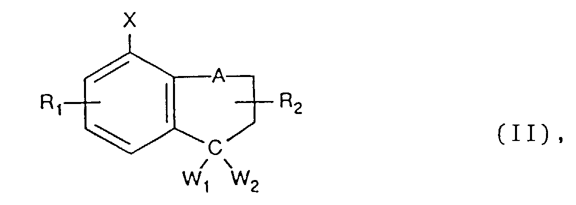 où le groupe CW₁W₂ représente un carbonyle, un carbonyle fonctionnellement modifié ou un carbonyle protégé et A, X, R₁ et R₂ ont les significations indiquées dans la revendication 1 pour les sels d'addition d'acides de formule I, avec une amine de formule 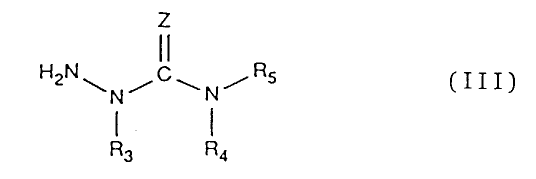 où Z, R₃, R₄ et R₅ ont les significations indiquées dans la revendication 1 pour les sels d'addition d'acides des bases de formule I, en présence d'un acide [PA] ayant les significations indiquées dans la revendication 1, ou
c) en ce que l'on transforme dans un composé de formule 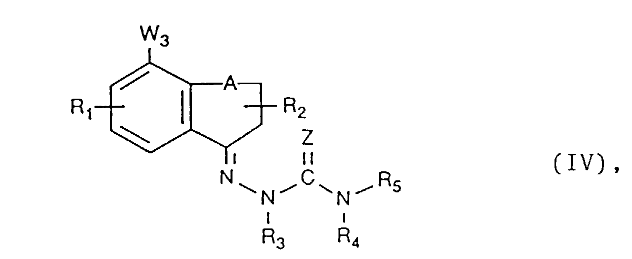 où W₃ représente un reste pouvant être transformé en un groupe X dans une base de formule I selon la revendication 1 et A, Z, R₁, R₂, R₃, R₄ et R₅ sont définis comme pour la formule I dans la revendication 1, le reste W₃ en groupe X, en présence d'un acide [PA] ayant les significations indiquées dans la revendication 1, ou
d) en ce que l'on transforme un sel d'addition d'acides quelconque d'une base de formule I avec un acide n'entrant pas dans la définition de [PA] dans la revendication 1, en un sel d'addition d'acides d'une base de formule I avec un acide [PA] ayant les significations indiquées dans la revendication 1 ;
et, si désiré, en ce que l'on transforme le sel d'addition d'acides d'une base de formule I obtenu avec un acide [PA] en un autre sel d'addition d'acides d'une base de formule I avec un acide [PA] et/ou, si désiré, en ce que l'on sépare les mélanges d'isomères en leurs isomères individuels.
